# EUROPEAN PATENT SPECIFICATION

(11) **EP 3 976 152 B1**
(45) Date of publication and mention of the grant of the patent: **15.05.2024**
(21) Application number: 20812976.7
(22) Date of filing: 28.05.2020
(51) Int. Cl.: A61M 16/20

(54) **VENT AND AAV ASSEMBLY**
ENLÜFTUNGS- UND AAV-ANORDNUNG
ENSEMBLE VENTILATION ET AAV

(30) Priority: 31.05.2019 US 201962855469 P
(43) Date of publication of application: 06.04.2022
(73) Proprietor: ResMed Pty Ltd, Bella Vista, New South Wales 2153 (AU)
(72) Inventor: DANTANARAYANA, Muditha Pradeep, Bella Vista, New South Wales 2153 (AU)
(74) Representative: Vossius & Partner Patentanwälte Rechtsanwälte mbB
(86) International application number: PCT/IB2020/055071
(87) International publication number: WO 2020/240462

(56) References cited:
- EP-A1- 3 202 449
- WO-A1-00/38772
- WO-A1-02/051486
- WO-A1-2016/041019
- WO-A1-2018/203759
- WO-A1-2019/119058
- WO-A1-2019/119058
- WO-A1-2020/170207
- US-A1- 2016 296 720
- US-A1- 2018 236 200
- US-A1- 2019 091 432

## Description

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO₂ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 2.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 2.2.3 Respiratory therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

WO 2019/119058 A1 discloses a patient interface comprising a plenum chamber, a seal-forming structure, a conduit being sized and structured to receive a flow of air at a therapeutic pressure, and a conduit connector configured to pneumatically connect the conduit to a corresponding plenum chamber hole to provide the flow of air at the therapeutic pressure to the patient interface plenum chamber.

US 2019/0091432 A1 discloses a patient interface comprising a plenum chamber, a seal-forming structure and an AAV.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.)*,* assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH₂O).

| RPT Device name | A-weighted sound pressure level dB(A) | Year (approx.) |
|---|---|---|
| C-Series Tango^{™} | 31.9 | 2007 |
| C-Series Tango^{™} with Humidifier | 33.1 | 2007 |
| S8 Escape^{™} II | 30.5 | 2005 |
| S8 Escape^{™} II with H4i^{™} Humidifier | 31.1 | 2005 |
| S9 AutoSet^{™} | 26.5 | 2010 |
| S9 AutoSet^{™} with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar^{™} Series of Adult and Paediatric Ventilators may provide support for invasive and non-invasive non-dependent ventilation for a range of patients for treating a number of conditions such as but not limited to NMD, OHS and COPD.

The ResMed Elisée^{™} 150 ventilator and ResMed VS III^{™} ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

WO 2018/203759 A1 discloses a conduit connector assembly for a respiratory therapy apparatus comprising a conduit having a first flow port configured to be connected to a patient interface, a second flow port configured to be connected to a gas delivery conduit and a supplementary flow port. The conduit connector assembly further comprises a hollow valve body configured to be mounted on or in the conduit and comprising at least a first valve port configured to be closed or opened by a valve flap, the conduit, valve body and the valve flap being configured to selectively provide two flow parts through the conduit connector assembly, being an inspiratory flow path from the second flow port to the first flow port, an expiratory flow path from the first flow port to the supplementary flow port via the first valve port, wherein a supplementary gas flow path is provided between the first and/or second flow ports and the supplementary flow port via a supplementary valve port of the valve body.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the start of inspiration. This therapy mode is known as pulsed or demand (oxygen) delivery (POD), in contrast with traditional continuous flow delivery more suited to stationary oxygen concentrators.

### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.7 Mandibular repositioning

A mandibular repositioning device (MRD) or mandibular advancement device (MAD) is one of the treatment options for sleep apnea and snoring. It is an adjustable oral appliance available from a dentist or other supplier that holds the lower jaw (mandible) in a forward position during sleep. The MRD is a removable device that a patient inserts into their mouth prior to going to sleep and removes following sleep. Thus, the MRD is not designed to be worn all of the time. The MRD may be custom made or produced in a standard form and includes a bite impression portion designed to allow fitting to a patient's teeth. This mechanical protrusion of the lower jaw expands the space behind the tongue, puts tension on the pharyngeal walls to reduce collapse of the airway and diminishes palate vibration.

In certain examples a mandibular advancement device may comprise an upper splint that is intended to engage with or fit over teeth on the upper jaw or maxilla and a lower splint that is intended to engage with or fit over teeth on the upper jaw or mandible. The upper and lower splints are connected together laterally via a pair of connecting rods. The pair of connecting rods are fixed symmetrically on the upper splint and on the lower splint.

In such a design the length of the connecting rods is selected such that when the MRD is placed in a patient's mouth the mandible is held in an advanced position. The length of the connecting rods may be adjusted to change the level of protrusion of the mandible. A dentist may determine a level of protrusion for the mandible that will determine the length of the connecting rods.

Some MRDs are structured to push the mandible forward relative to the maxilla while other MADs, such as the ResMed Narval CC^{™} MRD are designed to retain the mandible in a forward position. This device also reduces or minimises dental and temporo-mandibular joint (TMJ) side effects. Thus, it is configured to minimises or prevent any movement of one or more of the teeth.

### 2.2.3.8 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

WO 02/051486 A1 discloses a flow regulation vent for regulating flow from a pressurized gas supply. The vent is disposed in a housing and comprises a fixed portion adapted to engage a gas supply conduit, a spring force biased movable portion flowingly connected to the pressurized gas supply, and a hinge pivotally connecting the movable portion to the fixed portion. The fixed portion has a gas flow orifice and the movable portion is pivotally movable between (1) a relaxed position, whereby at a specified minimum operating pressure, the movable portion is pivoted by the spring force to a position away from the fixed portion to establish a first gas flow area between the movable portion and the gas flow orifice, and (2) a fully pressurized position, whereby, at a specified operating pressure, the pressurized gas offsets the spring force to pivot the movable portion to a position adjacent the fixed portion to establish a minimum gas flow area between the movable portion and the gas flow orifice.

Table of noise of prior masks (ISO 17510-2:2007, 10 cmH₂O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage^{™} (*) | nasal | 29.5 | 21.5 | 1998 |
| ResMed UltraMirage^{™} | nasal | 36 (3) | 28 (3) | 2000 |
| ResMed Mirage Activa^{™} | nasal | 32 (3) | 24 (3) | 2002 |
| ResMed Mirage Micro^{™} | nasal | 30 (3) | 22 (3) | 2008 |
| ResMed Mirage^{™} SoftGel | nasal | 29 (3) | 22 (3) | 2008 |
| ResMed Mirage^{™} FX | nasal | 26(3) | 18 (3) | 2010 |
| ResMed Mirage Swift^{™} (*) | nasal pillows | 37 | 29 | 2004 |
| ResMed Mirage Swift^{™} II | nasal pillows | 28 (3) | 20 (3) | 2005 |
| ResMed Mirage Swift^{™} LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of the present technology relates to a patient interface including a vent to discharge gas exhaled by the patient. The patient interface may comprise a diffusing member structured and arranged to diffuse the vent flow to produce less noise. The patient interface may include an anti-asphyxia valve (AAV) configured to allow the patient to breathe in ambient air and exhale if pressurized gas is not of sufficient magnitude or not delivered.

Another aspect of the present technology relates to a patient interface to deliver a flow of air at a positive pressure with respect to ambient air pressure to an entrance to the patient's airways including at least the entrance of a patient's nares while the patient is sleeping, to ameliorate sleep disordered breathing. The patient interface includes a seal-forming structure constructed and arranged to form a seal with a region of a patient's face surrounding the entrance to the patient's airways, the seal-forming structure forming at least a portion of a plenum chamber pressurizable to a therapeutic pressure and a vent and AAV assembly. The vent and AAV assembly is configured to regulate flow therethrough to (1) provide a vent flow path when pressure in the plenum chamber is above a predetermined magnitude and (2) provide a breathable flow path when pressure in the plenum chamber is below the predetermined magnitude or not delivered. The vent and AAV assembly includes a vent and AAV housing and an AAV member provided to the vent and AAV housing. The vent and AAV housing includes at least one first opening and at least one second opening extending therethrough, each of the at least one first opening and the at least one second opening configured to allow gas to flow between the plenum chamber and ambient. The AAV member includes at least one flap portion structured and arranged to regulate flow through the at least one first opening and the at least one second opening. The at least one flap portion includes a plurality of vent holes therethrough. The at least one flap portion is movable to an activated position when pressure in the plenum chamber is above the predetermined magnitude to cover the at least one first opening and the at least one second opening so that a vent flow of gas is allowed to pass along the vent flow path that extends through the plurality of vent holes of the at least one flap portion and through the at least one second opening, and the at least one flap portion is movable to a deactivated position when pressure in the plenum chamber is below the predetermined magnitude or not delivered to uncover the at least one first opening and the at least one second opening so that a breathable flow of gas is allowed to pass along the breathable flow path that extends through the at least one first opening and the at least one second opening.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a patient interface that may be washed in a home of a patient, e.g., in soapy water, without requiring specialised cleaning equipment.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

The present invention is defined by the appended claims.

### 4 BRIEF DESCRIPTION OF THE DRAWINGS

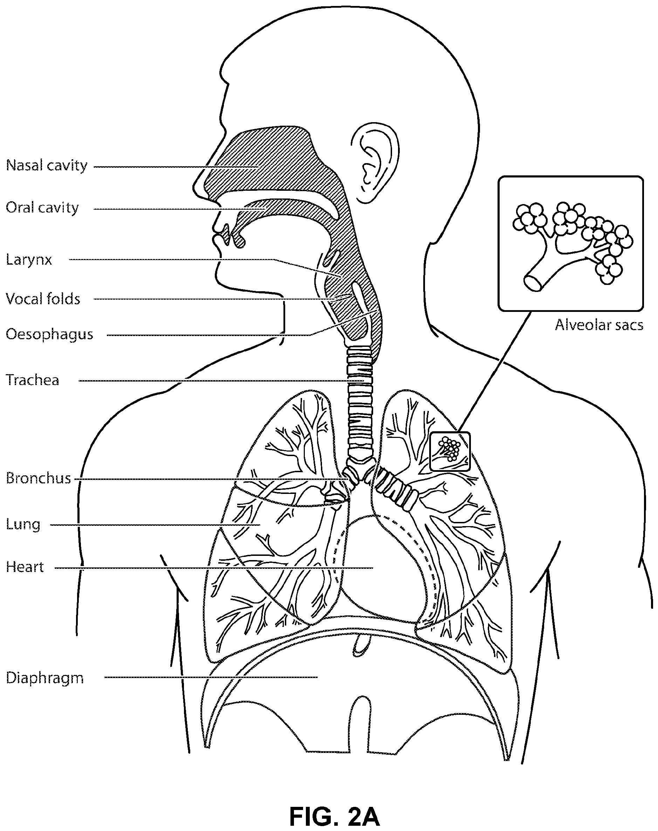

The present technology is illustrated by way of example, and not by way of limitation, in the figures of the accompanying drawings, in which like reference numerals refer to similar elements including:
4.1 RESPIRATORY THERAPY SYSTEMS
   Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.
   Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
   Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.
4.2 RESPIRATORY SYSTEM AND FACIAL ANATOMY
   Fig. 2A shows an overview of a human respiratory system including the nasal and oral cavities, the larynx, vocal folds, oesophagus, trachea, bronchus, lung, alveolar sacs, heart and diaphragm.

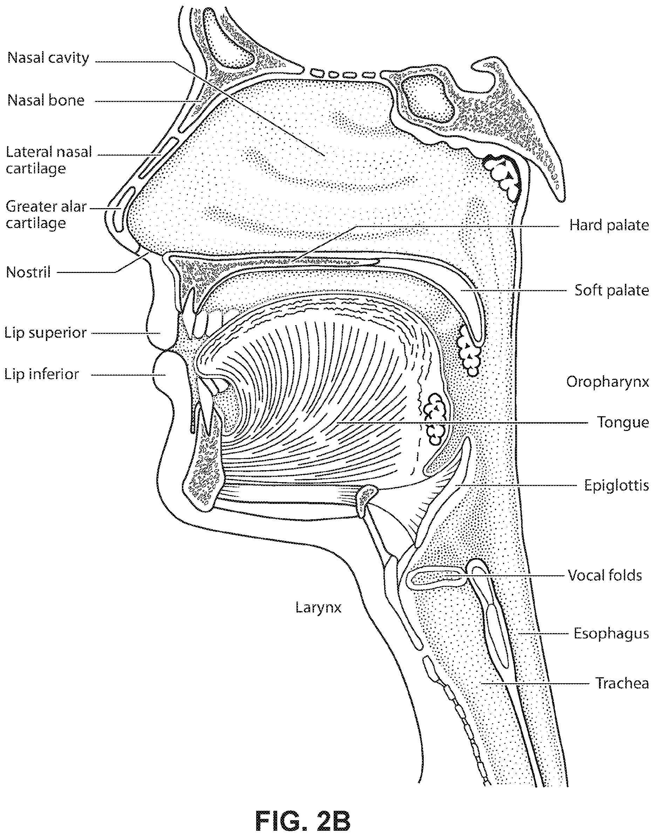

   Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

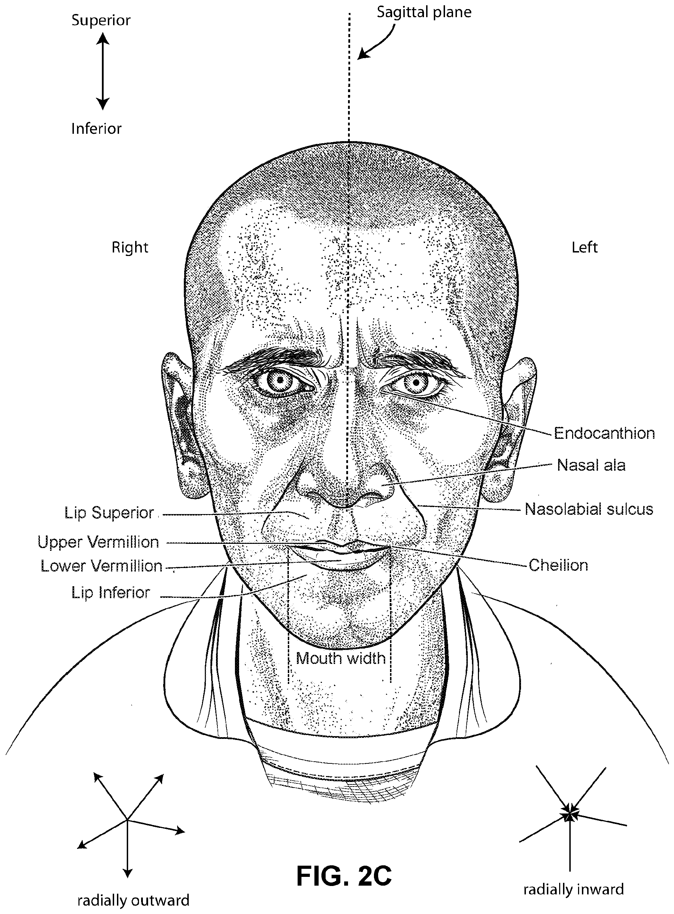

   Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

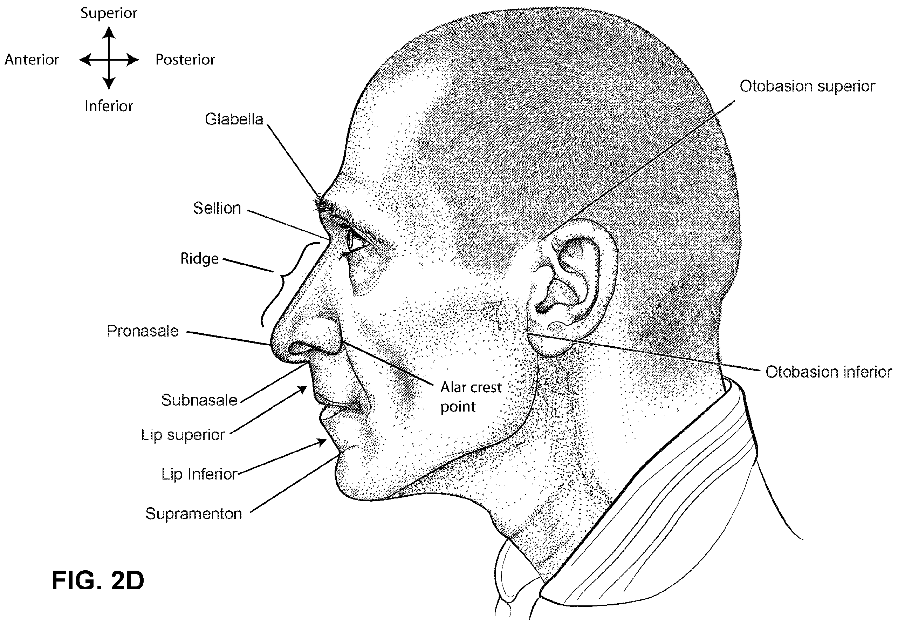

   Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

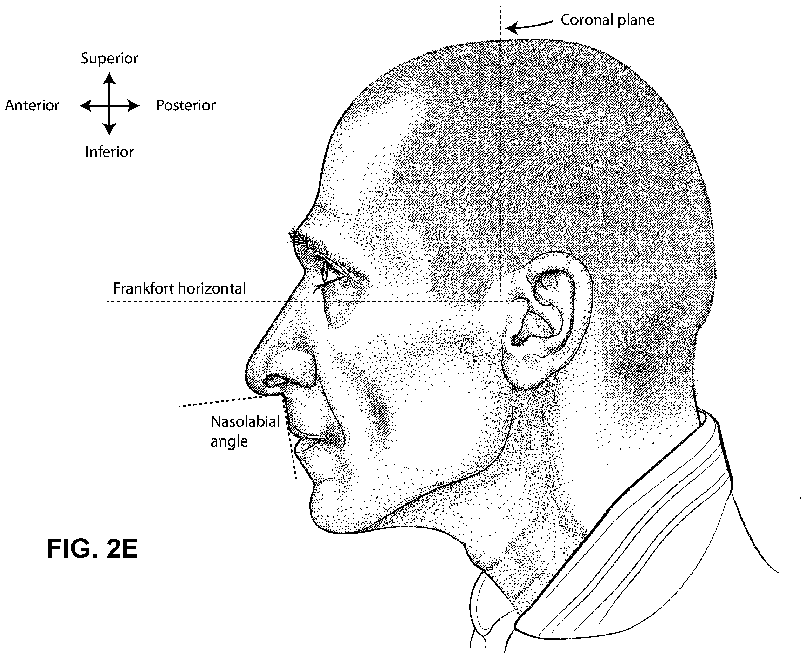

   Fig. 2E is a further side view of a head. The approximate locations of the Frankfort horizontal and nasolabial angle are indicated. The coronal plane is also indicated.

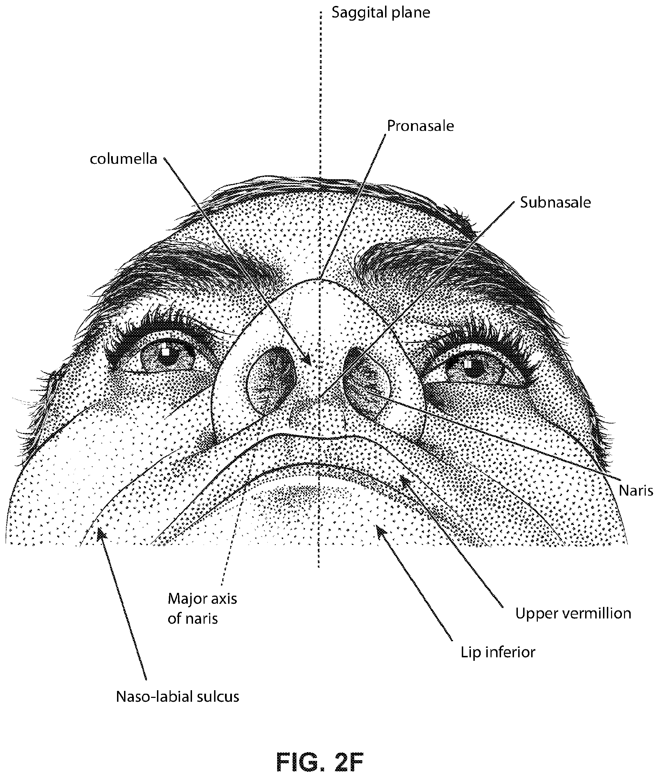

   Fig. 2F shows a base view of a nose with several features identified including naso-labial sulcus, lip inferior, upper Vermilion, naris, subnasale, columella, pronasale, the major axis of a naris and the midsagittal plane.

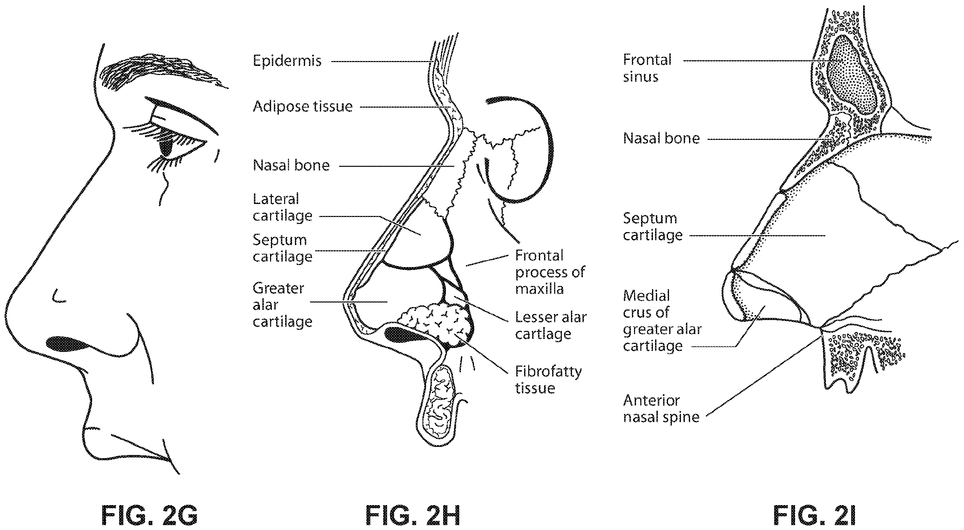

   Fig. 2G shows a side view of the superficial features of a nose.
   Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
   Fig. 2I shows a medial dissection of a nose, approximately several millimeters from the midsagittal plane, amongst other things showing the septum cartilage and medial crus of greater alar cartilage.

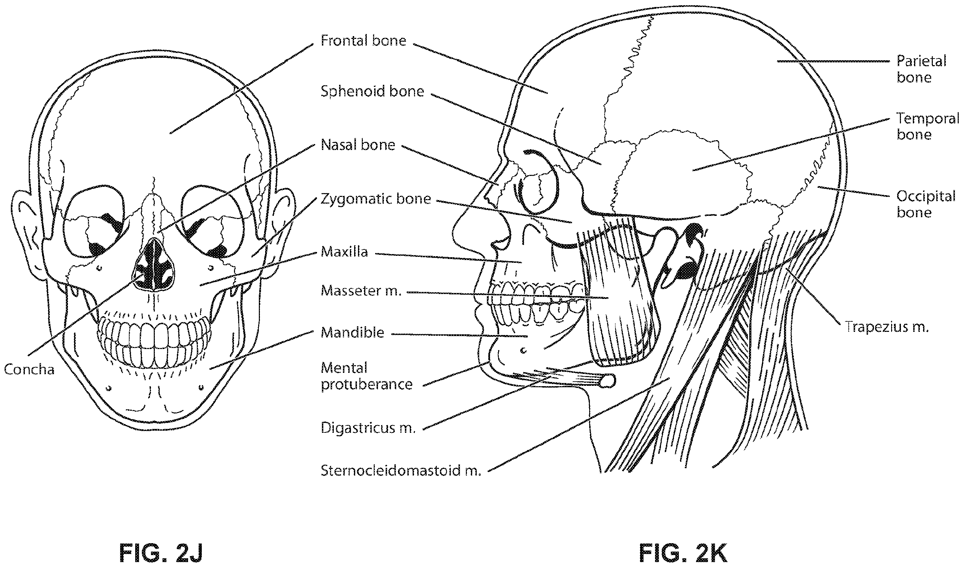

   Fig. 2J shows a front view of the bones of a skull including the frontal, nasal and zygomatic bones. Nasal concha are indicated, as are the maxilla, and mandible.
   Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

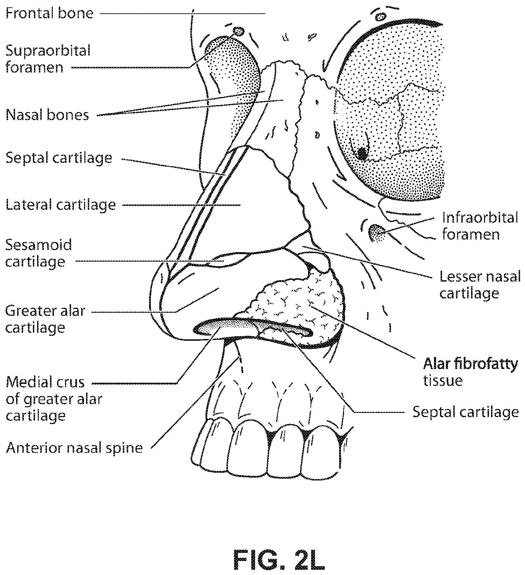

   Fig. 2L shows an anterolateral view of a nose.
4.3 PATIENT INTERFACE
   Fig. 3A shows a patient interface in the form of a nasal mask in accordance with one form of the present technology.
   Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
   Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
   Fig. 3D shows a schematic of a cross-section through a structure at a point. An outward normal at the point is indicated. The curvature at the point has a value of zero.
   Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
   Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
   Fig. 3G shows a cushion for a mask that includes two pillows. An exterior surface of the cushion is indicated. An edge of the surface is indicated. Dome and saddle regions are indicated.
   Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
   Fig. 3I shows the surface of a structure, with a one dimensional hole in the surface. The illustrated plane curve forms the boundary of a one dimensional hole.
   Fig. 3J shows a cross-section through the structure of Fig.3I. The illustrated surface bounds a two dimensional hole in the structure of Fig. 3I.
   Fig. 3K shows a perspective view of the structure of Fig. 3I, including the two dimensional hole and the one dimensional hole. Also shown is the surface that bounds a two dimensional hole in the structure of Fig. 3I.
   Fig. 3L shows a mask having an inflatable bladder as a cushion.
   Fig. 3M shows a cross-section through the mask of Fig. 3L, and shows the interior surface of the bladder. The interior surface bounds the two dimensional hole in the mask.
   Fig. 3N shows a further cross-section through the mask of Fig. 3L. The interior surface is also indicated.
   Fig. 3O illustrates a left-hand rule.
   Fig. 3P illustrates a right-hand rule.
   Fig. 3Q shows a left ear, including the left ear helix.
   Fig. 3R shows a right ear, including the right ear helix.
   Fig. 3S shows a right-hand helix.
   Fig. 3T shows a view of a mask, including the sign of the torsion of the space curve defined by the edge of the sealing membrane in different regions of the mask.
   Fig. 3U shows a view of a plenum chamber 3200 showing a sagittal plane and a mid-contact plane.
   Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

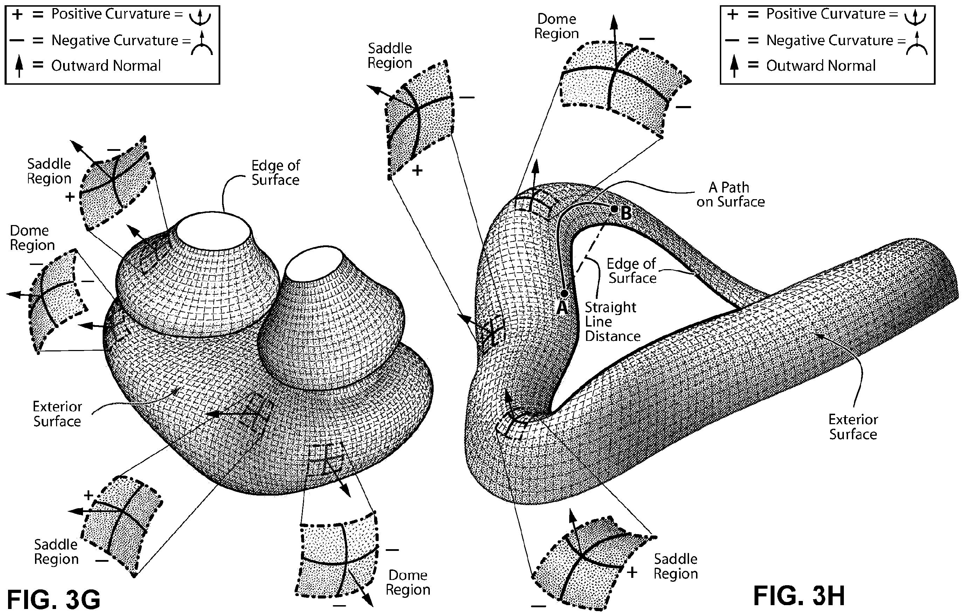

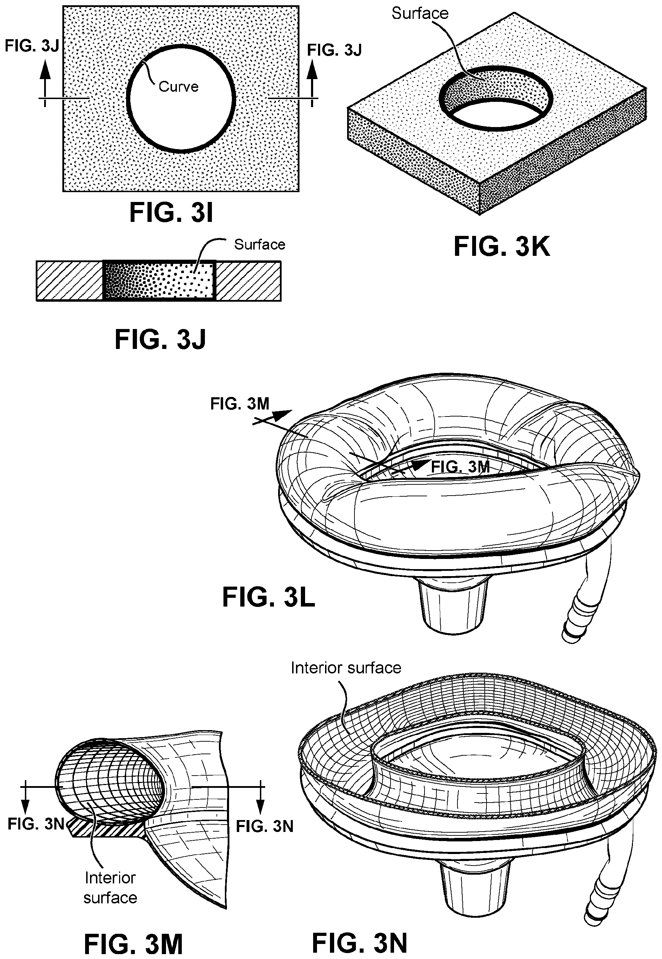

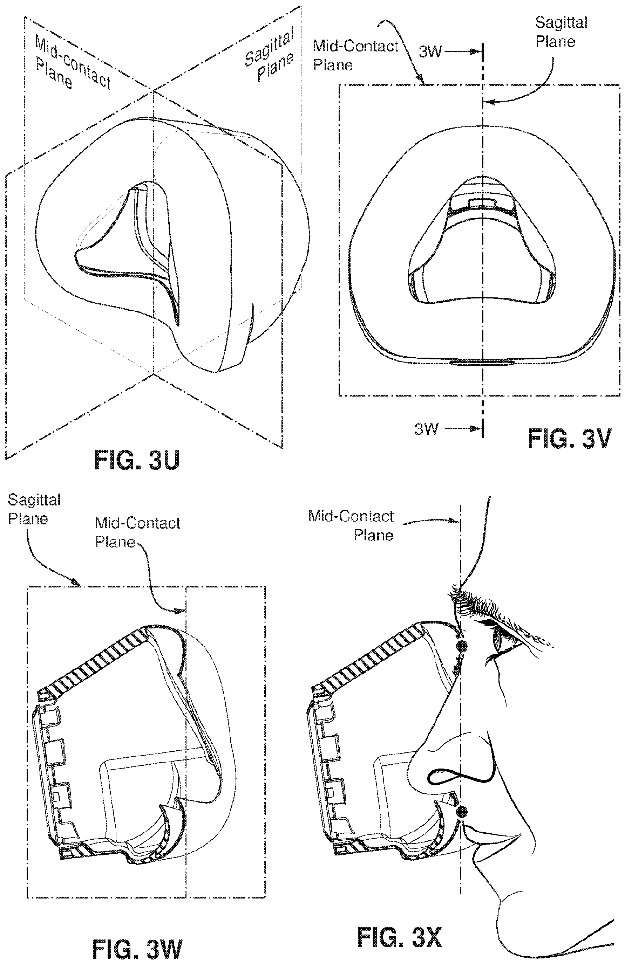

   Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
   Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the `plane of the face' when the plenum chamber is in position for use. In Fig. 3X the plenum chamber 3200 is that of a nasal mask, and the superior point 3220 sits approximately on the sellion, while the inferior point 3230 sits on the lip superior.
   Fig. 4 shows a patient interface in accordance with another form of the present technology.
4.4 VENT AND AAV ASSEMBLY
   Fig. 5A is a perspective view of a vent and AAV assembly provided to a shell of a patient interface according to an example of the present technology.
   Fig. 5B is an exploded view of a vent and AAV assembly and shell of a patient interface according to an example of the present technology.
   Fig. 5C is another exploded view of a vent and AAV assembly and shell of a patient interface according to an example of the present technology.
   Fig. 5D is a perspective view of a vent and AAV assembly according to an example of the present technology.
   Fig. 5E is another perspective view of the vent and AAV assembly of Fig. 5D.
   Fig. 5F is a cross-sectional view of the vent and AAV assembly of Fig. 5D.
   Fig. 5G is another cross-sectional view of the vent and AAV assembly of Fig. 5D.
   Fig. 5H is an exploded view of the vent and AAV assembly of Fig. 5D.
   Fig. 5I is another exploded view of the vent and AAV assembly of Fig. 5D.
   Fig. 5J is a perspective view of a vent and AAV housing of the vent and AAV assembly of Fig. 5D.
   Fig. 5K is another perspective view of the vent and AAV housing of Fig. 5J.
   Fig. 5L is a perspective view of a diffusing member cover of the vent and AAV assembly of Fig. 5D.
   Fig. 5M is another perspective view of the diffusing member cover of Fig. 5L.
   Fig. 5N is a perspective view of an AAV member of the vent and AAV assembly of Fig. 5D.
   Fig. 5O is another perspective view of the AAV member of Fig. 5N.
   Fig. 5P is a perspective view of an AAV cover of the vent and AAV assembly of Fig. 5D.
   Fig. 5Q is a cross-sectional view showing air passing through the vent and AAV assembly of Fig. 5D when pressure in the patient interface is below a predetermined magnitude or not delivered according to an example of the present technology.
   Fig. 5R is a cross-sectional view showing air passing through the vent and AAV assembly of Fig. 5D when pressure in the patient interface is above a predetermined magnitude according to an example of the present technology.
   Fig. 5S is an exploded view of a vent and AAV assembly according to an example of the present technology.
   Fig. 5T is another exploded view of the vent and AAV assembly of Fig. 5S.
   Fig. 5U is a cross-sectional view of a vent and AAV assembly according to an example of the present technology.
   Fig. 5V is another cross-sectional view of the vent and AAV assembly of Fig. 5U.
   Fig. 5W is a perspective view of an AAV member and AAV cover of the vent and AAV assembly according to an example of the present technology.
   Fig. 6 is a perspective view of an AAV member according to another example of the present technology.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

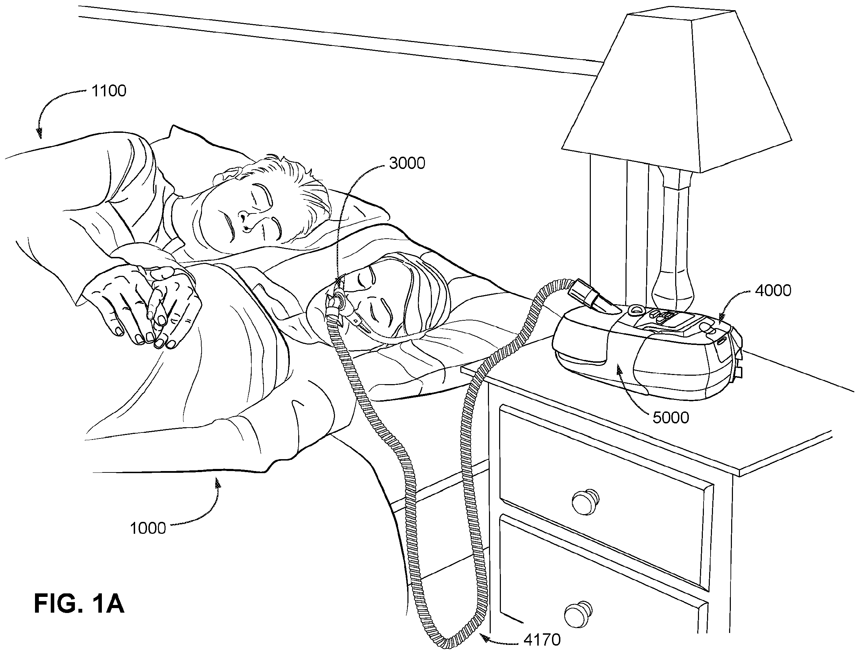

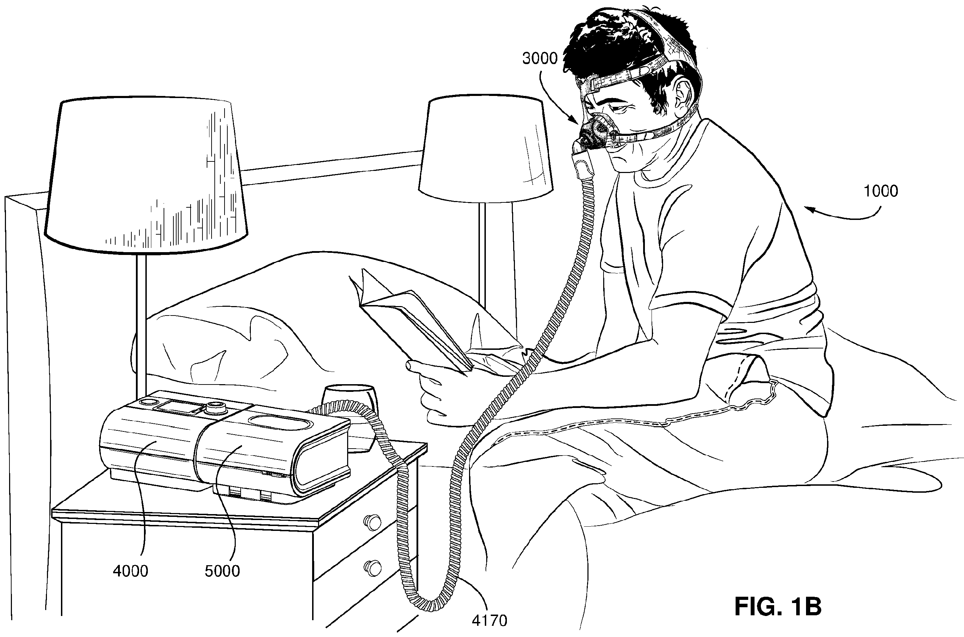

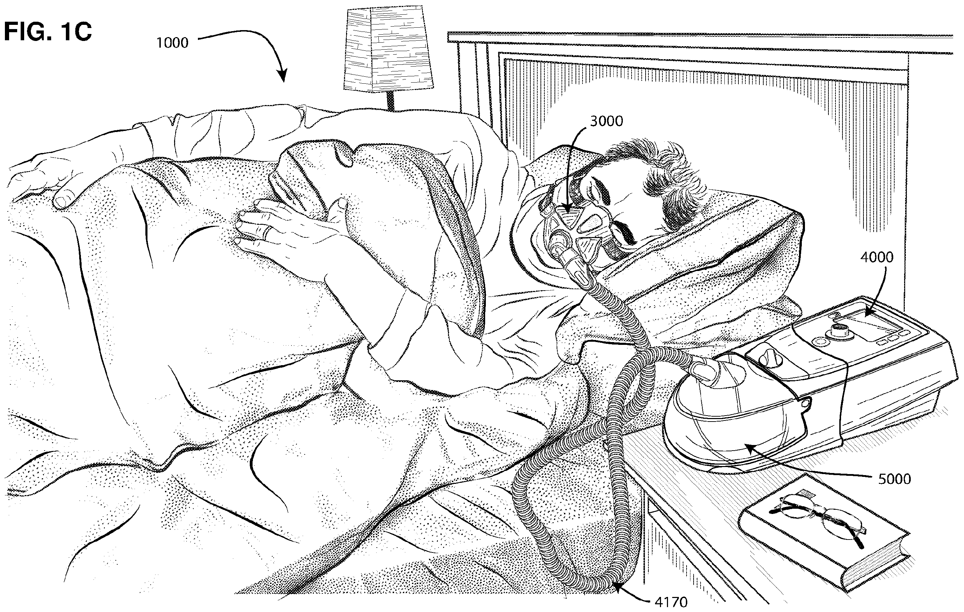

In one form, the present technology comprises a respiratory therapy system for treating a respiratory disorder. The a respiratory therapy system may comprise an RPT device 4000 for supplying a flow of air to the patient 1000 via an air circuit 4170 and a patient interface 3000, e.g., see Figs. 1A to 1C.

### 5.3 PATIENT INTERFACE

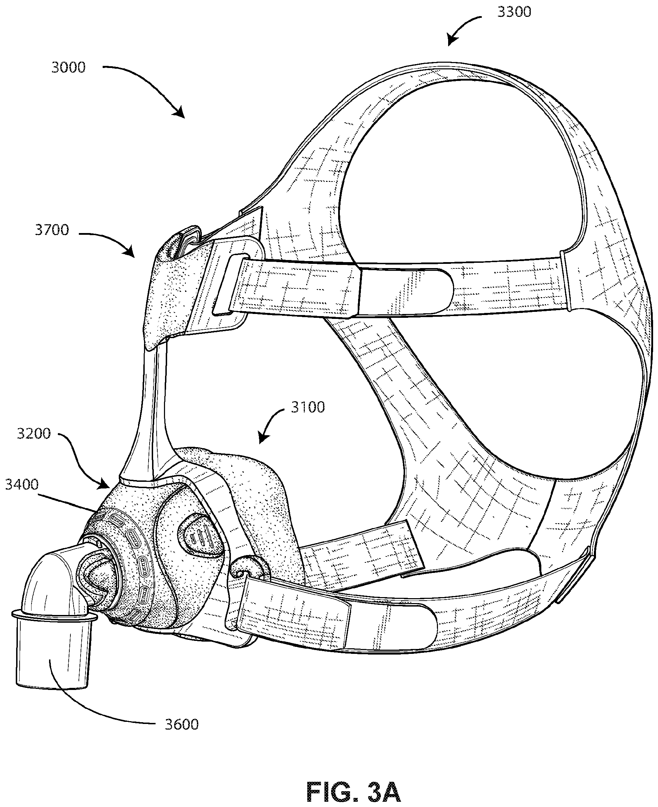

As shown in Fig. 3A, a non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

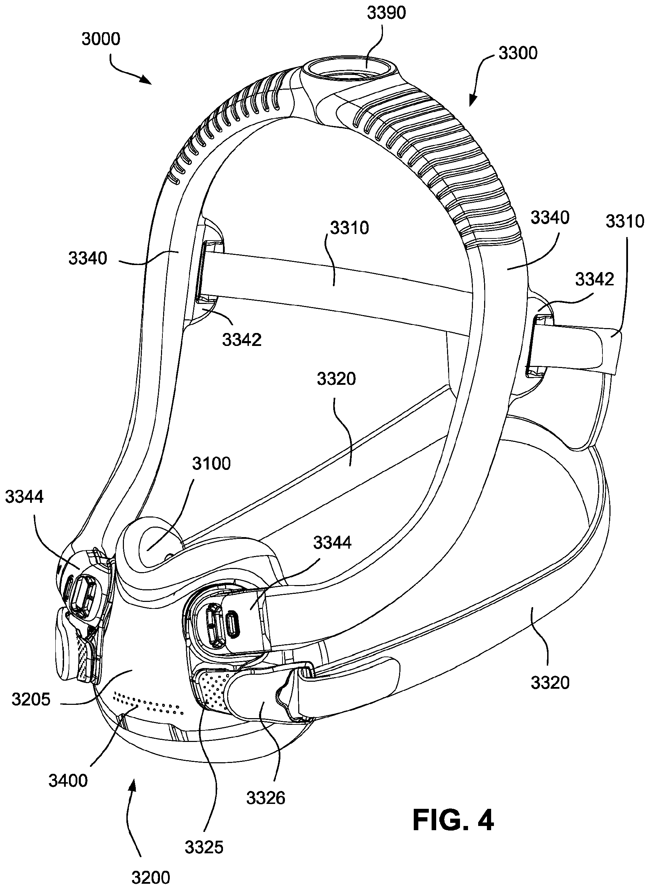

Fig. 4 shows a patient interface 3000 in accordance with another form of the present technology. The patient interface 3000 in this example also comprises a positioning and stabilising structure 3300 to hold the plenum chamber 3200 in sealing position on the patient's face in use. The positioning and stabilising structure 3300 in this example comprises a pair of headgear tubes 3340. The pair of headgear tubes 3340 are connected to each other at their superior ends and are each configured to lie against superior and lateral surfaces of the patient's head in use. Each of the headgear tubes 3340 may be configured to lie between and eye and an ear of the patient in use. The inferior end of each headgear tube 3340 is configured to fluidly connect to the plenum chamber 3200. In this example, the inferior end of each headgear tube 3340 connects to a headgear tube connector 3344 configured to connect to the shell 3205 of the plenum chamber 3200. The positioning and stabilising structure 3300 comprises a conduit headgear inlet 3390 at the junction of the two headgear tubes 3340. The conduit headgear inlet 3390 is configured to receive a pressurised flow of gas, for example via an elbow comprising a connection port 3600, and allow the flow of gas into hollow interiors of the headgear tubes 3340. The headgear tubes 3340 supply the pressurised flow of gas to the plenum chamber 3200.

The positioning and stabilising structure 3300 may comprise one or more straps in addition to the headgear tubes 3340. In this example the positioning and stabilising structure 3300 comprises a pair of upper straps 3310 and a pair of lower straps 3320. The posterior ends of the upper straps 3310 and lower straps 3320 are joined together. The junction between the upper straps 3310 and lower strap 3320 is configured to lie against a posterior surface of the patient's head in use, providing an anchor for the upper strap 3310 and lower straps 3320. Anterior ends of the upper straps 3310 connect to the headgear tubes 3340. In this example each headgear tube 3340 comprises a tab 3342 having an opening through which a respective upper strap 3310 can be passed through and then looped back and secured onto itself to secure the upper headgear strap 3310 to the headgear tube 3340. The positioning and stabilising structure 3300 also comprises a lower strap clip 3326 provided to the anterior end of each of the lower straps 3320. Each of the lower strap clip 3326 is configured to connect to a lower connection point 3325 on the plenum chamber 3200. In this example, the lower strap clips 3326 are secured magnetically to the lower connection points 3325. In some examples, there is also a mechanical engagement between the lower strap clips 3326 and the lower connection points 3325.

In some examples of the present technology, the plenum chamber 3200 is at least partially formed by the shell 3205 and the seal-forming structure 3100. The plenum chamber 3200 may comprise a cushion module or cushion assembly, for example. The shell 3205 may function as a chassis for the seal-forming structure 3100.

The exemplary patient interface 3000 in Fig. 4 is an oronasal patient interface. That is, the patient interface 3000 is configured to seal around both the patient's nasal airways and oral airway. In some examples the patient interface 3000 comprises separate seals around each of the nasal airways and oral airway. The patient interface 3000 may comprise a plenum chamber 3200 having a nasal portion and an oral portion. The seal forming structure may be configured to surround the nasal airways at the nasal portion and to seal around the patient's mouth at the oral portion. As such, the seal-forming structure 3100 may also be considered to have a nasal portion and an oral portion, the nasal portions and oral portions of the seal-forming structure comprising those parts that seal around the patient's nasal airways and mouth respectively.

In an example, the seal-forming structure 3100 at the nasal portion does not lie over a nose bridge region or nose ridge region of the patient's face and instead seals against inferior surfaces of the patient's nose. The nasal portion may seal against the lip superior, the ala and the anterior surface of the pronasale and/or the inferior surface of the pronasale. The actual sealing locations may differ between patients. The nasal portion may also be configured to contact and/or seal to a region of the patient's face between the ala and the nasolabial sulcus and at the lateral portions of the lip superior proximate the nasolabial sulcus.

The seal-forming structure 3100 of the oral portion may be configured to form a seal to a periphery of the patient's mouth in use. The oral portion may be configured to form a seal to the patient's face at the lip superior, nasolabial sulcus, cheeks, lip inferior, supramenton, for example.

The seal-forming structure 3100 may have one or more holes therein such that the flow of air at a therapeutic pressure is delivered to the patient's nares and to the patient's mouth via the one or more holes. The seal-forming structure may define an oral hole and one or more nasal holes to deliver the flow of air to the patient. In an example, the plenum chamber 3200 comprises a seal-forming structure 3100 comprising an oral hole and two nasal holes. Each of the nasal holes may be positioned on the plenum chamber 3200 to be substantially aligned with a nare of the patient in order to deliver a flow of air thereto in use.

Further examples and details of the oronasal patient interface of Fig. 4 are described in PCT Application No. PCT/AU2019/050278, filed March 28, 2019.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.5 Forehead region

In one form, the seal-forming structure that forms a seal in use on a forehead region of the patient's face. In such a form, the plenum chamber may cover the eyes in use.

### 5.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 5.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### Vent and AAV Assembly

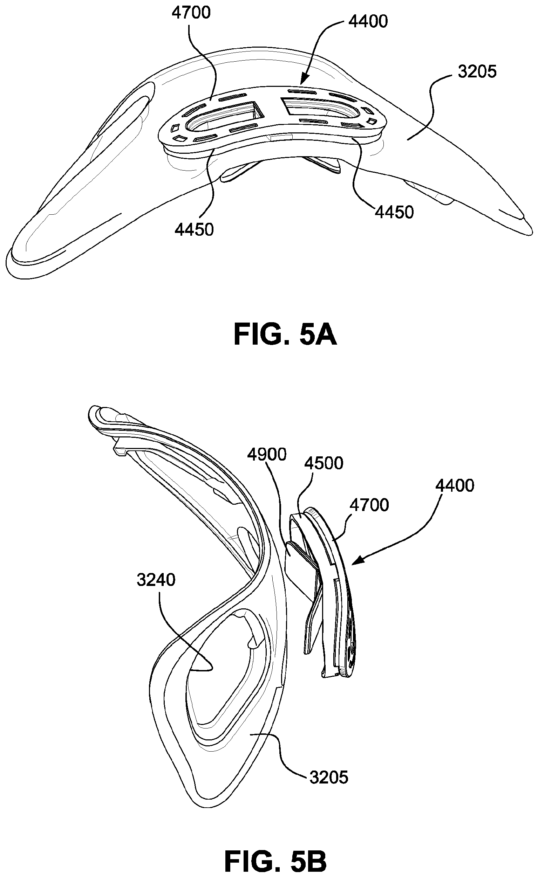

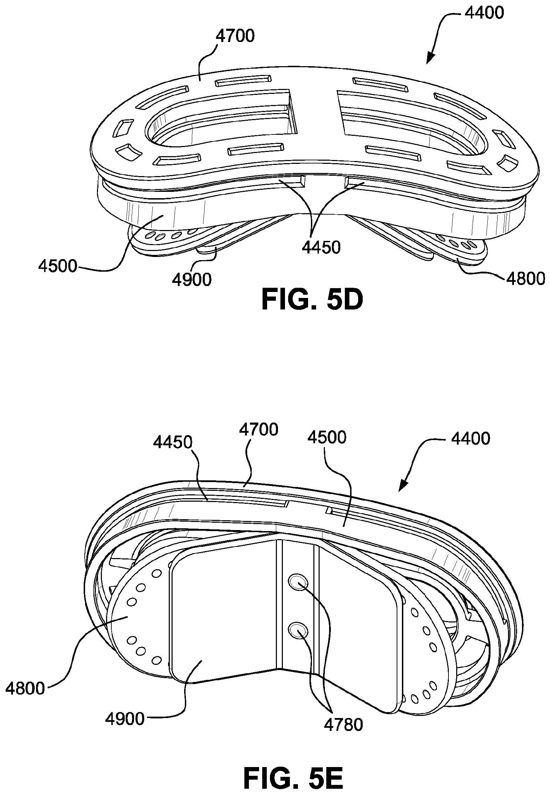

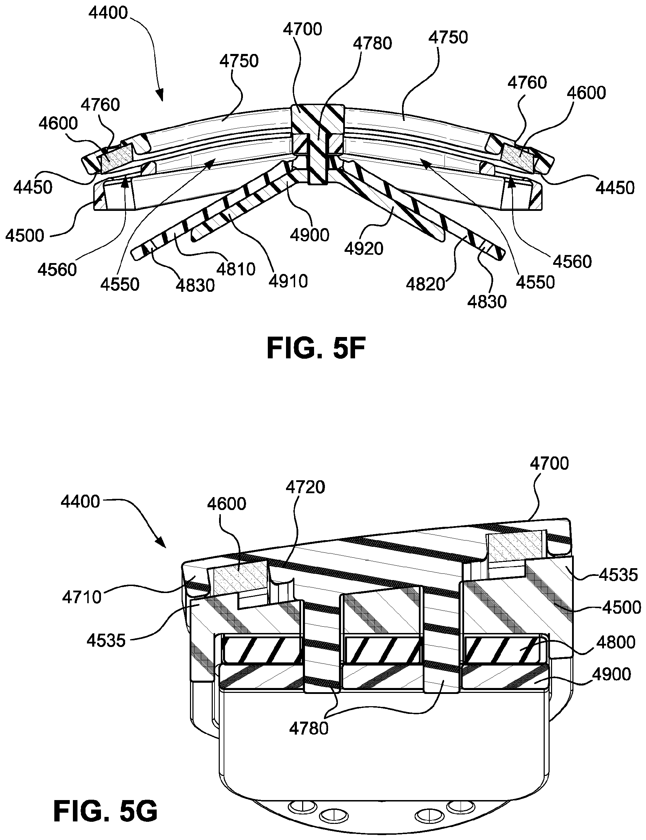

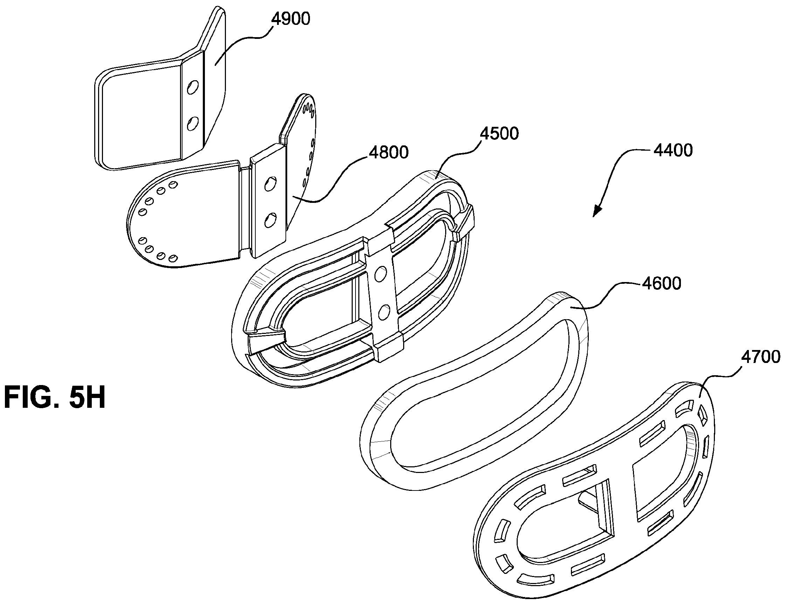

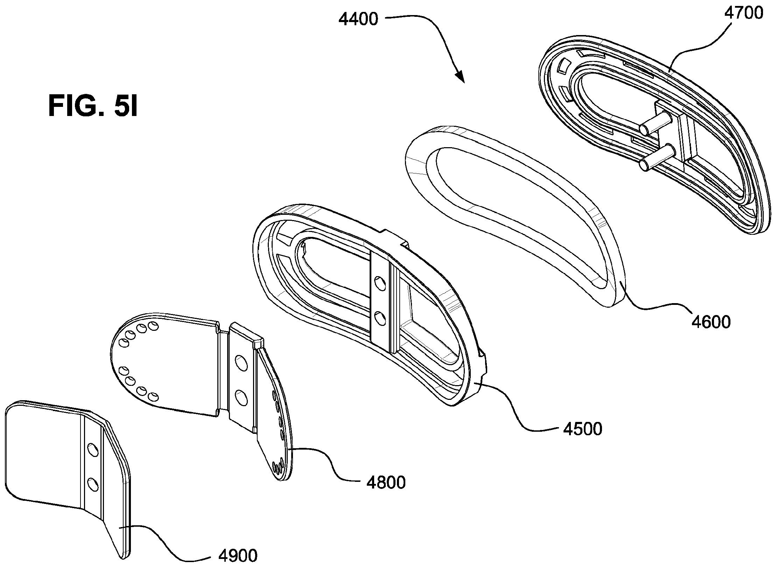

Figs. 5A to 5W show a vent and anti-asphyxia valve (AAV) assembly 4400 according to an example of the present technology. The vent and AAV assembly 4400 is configured to provide a vent flow of gas to discharge gas exhaled by the patient. In the illustrated example, the vent and AAV assembly 4400 comprises a diffusing member 4600 along the vent flow path structured and arranged to diffuse the exhaust vent flow to produce less noise. In addition, the vent and AAV assembly 4400 is configured to allow the patient to breathe in ambient air and exhale through one or more openings if pressurized gas is not of sufficient magnitude or not delivered.

In the illustrated example, as best shown in Figs. 5A to 5C, the vent and AAV assembly 4400 is in the form of a vent insert or vent module structured to be inserted into an opening 3250 in a patient interface 3000.

In the illustrated example, the patient interface 3000 is an oronasal patient interface, e.g., such as the type shown in Fig. 4. However, it should be appreciated that aspects of the present technology may be adapted for use with other suitable interface types.

In illustrated example, the shell or chassis 3205 of the oronasal patient interface comprises two inlet ports 3240 provided to lateral sides of the shell 3205. The inlet ports 3240 in this example are configured to connect to respective ones of the headgear tubes 3340. In an example, the inlet ports 3240 may receive combined headgear and conduit connection assemblies (e.g., headgear tube connector 3344 as shown in Fig. 4) in order to provide multiple functions such as supply of the flow of air and headgear attachment points.

In the illustrated example, the shell or chassis 3205 comprises an opening 3250 adapted to receive the vent and AAV assembly 4400. The opening 3250 is provided centrally with respect to the plenum chamber 3200, e.g., so that that the vent and AAV assembly 4400 is provided centrally and less prone to being blocked during side sleeping. Additionally, the opening 3250 is provided at an inferior location on the shell 3205, e.g., so that the vent and AAV assembly 4400 is aligned approximately with the patient's mouth which provides a large portion of inhaled/exhaled gas. Furthermore, since the inlet ports 3240 of the plenum chamber 3200 are provided at a superior location on the plenum chamber 3200, a bias flow of air received at the inlet ports 3240 may flow through a large volume (e.g. from a superior location to an inferior location), which may provide for efficient gas washout and may reduce the likelihood of stagnant air pockets bypassed by the bias flow.

The vent and AAV assembly 4400 may be removably or permanently secured within the opening 3250 in any suitable manner, e.g., press fit assembly, snap or interference fit assembly (e.g., vent and AAV assembly 4400 may include a groove around its periphery, the groove adapted to locate the vent and AAV assembly 4400 against a correspondingly sized rim of the opening 3250 of the shell 3205), adhesive. In an alternative example, one or more portions of the vent and AAV assembly 4400 may be formed in one piece with the shell 3205, e.g., by over-molding or insert-molding.

In the illustrated example, the vent and AAV assembly 4400 and corresponding opening 3250 in the shell 3205 includes a stadium-shape, i.e., a rectangle with semicircles at opposite sides. However, it should be appreciated the vent and AAV assembly and corresponding opening in the shell may have other suitable shapes, e.g., circular and non-circular shapes.

In use, the vent and AAV assembly 4400 is structured and arranged to allow gas flow between an interior of the patient interface, e.g., the plenum chamber, and an exterior of the patient interface, e.g., atmosphere.

As shown in the example of Figs. 5D to 5P, the vent and AAV assembly 4400 comprises a vent and AAV housing 4500, a diffusing member 4600 provided to the vent and AAV housing 4500, a diffusing member cover 4700 to maintain the diffusing member 4600 to the vent and AAV housing 4500, an AAV member 4800 provided to the vent and AAV housing 4500, and an AAV cover 4900 to maintain the AAV member 4800 to the vent and AAV housing 4500. In use, the AAV member 4800 is structured and arranged to regulate air flow through the vent and AAV assembly 4400 and to provide sufficient washout of gas in use.

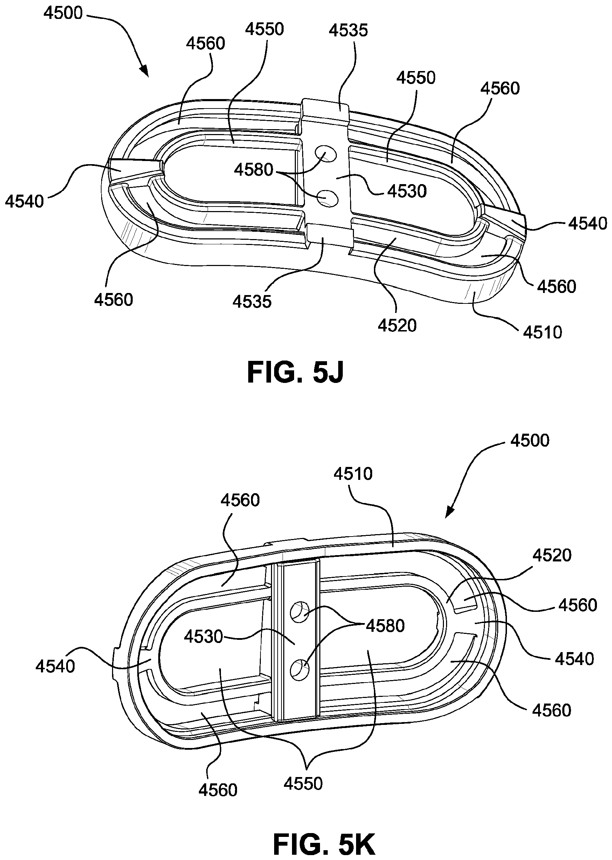

In the illustrated example, e.g., see Figs. 5J and 5K, the vent and AAV housing 4500 comprises a stadium-shaped (i.e., a rectangle with semicircles at opposite sides) outer wall 4510, a stadium shaped inner wall 4520, a base member 4530 extending along the minor axis across the outer and inner walls 4510, 4520, and support members 4540 extending along the major axis between the outer and inner walls 4510, 4520.

As illustrated, the base member 4530 bisects the inner wall 4520 to form a pair of inner openings 4550 (i.e., a first inner opening and a second inner opening), and the base member 4530 and the support members 4540 extend between the outer and inner walls 4510, 4520 to form outer openings 4560 that extend around the inner openings 4550 (i.e., a first pair of outer openings around the first inner opening and a second pair of outer openings around the second inner opening).

The base member 4530 and the support members 4540 are also structured and arranged to support the diffusing member 4600 along an anterior side of the vent and AAV housing 4500. As illustrated, a support 4535 is provided to each end of the base member 4530 that protrudes beyond anterior sides of the outer and inner walls 4510, 4520. Also, the support members 4540 between the outer and inner walls 4510, 4520 are arranged to protrude beyond anterior sides of the outer and inner walls 4510, 4520.

The diffusing member 4600 includes comprises a stadium-loop shape which forms an opening 4610 through the diffusing member 4600. The diffusing member 4600 is supported along the anterior side of the vent and AAV housing 4500 by the supports 4535 of the base member 4530 along its minor axis and by the support members 4540 along its major axis. The supports 4535 and the support members 4540 provide anterior surfaces (oriented towards atmosphere in use) to support the diffusing member 4600.

As illustrated, the diffusing member 4600 is arranged to cover the anterior ends of the outer openings 4560 of the vent and AAV housing 4500 so that at least a portion of the flow entering and/or exiting the outer openings 4560 can flow into the diffusing member 4600. Moreover, the opening 4610 of the diffusing member 4600 is arranged to align with the inner openings 4550 of the vent and AAV housing 4500 so that the inner openings 4550 are not covered by the diffusing member 4600 and flow entering and/or exiting the inner openings 4550 will not flow into the diffusing member 4600. Further, the supports 4535 and the support members 4540 are arranged so that the diffusing member 4600 is supported in spaced relation from the anterior ends of the the outer openings 4560, so at that at least a portion of the flow entering and/or exiting the outer openings 4560 can bypass the diffusing member 4600 and flow through lateral openings 4450 formed between the diffusing member 4600 and the vent and AAV housing 4500.

In an example, the diffusing member 4600 may be constructed of a porous material that allows gas to flow through the material but diffuses any jet or other flow formation entering and/or exiting the outer openings 4560, e.g., textile material such as a non-woven fibrous material or a woven fibrous material. In an example, the diffusing member 4600 may comprise a diffusing material which may be similar to or the same as a filter media. In an example, the diffusing member 4600 may comprise a thickness of about 0.1-0.5 mm, e.g., about 0.25 mm, however other suitable thicknesses are possible. In the illustrated example, the diffusing member 4600 comprises a single layer, however it should be appreciated that the diffusing member 4600 may comprise two or more layers, e.g., stacked layers, of similar or dissimilar diffusing materials.

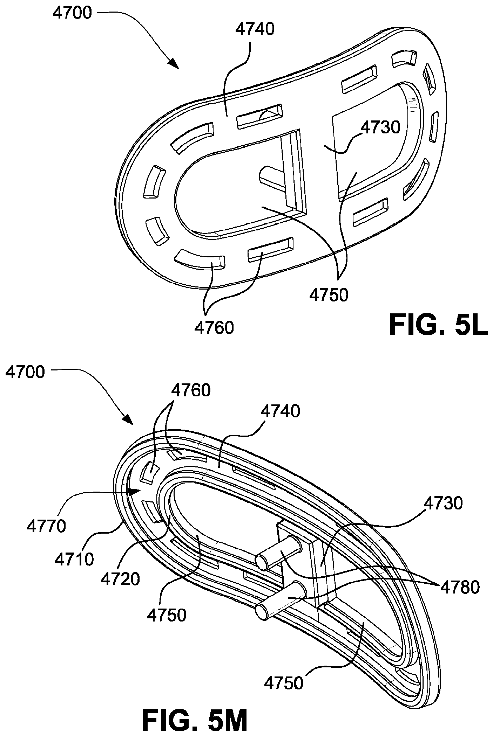

As best shown in Figs. 5L and 5M, the diffusing member cover 4700 comprises a stadium-shaped outer wall 4710, a stadium shaped inner wall 4720, a base member 4730 extending along the minor axis across the inner wall 4720, and an anterior wall 4740 extending along the perimeter of the cover 4700 between the outer and inner walls 4710, 4720. A plurality of spaced-apart slots or openings 4760 are formed through the anterior wall 4740. Also, the base member 4730 bisects the inner wall 4710 to form a pair of inner openings 4750 (i.e., a first inner opening and a second inner opening).

The outer wall 4710, inner wall 4720, and anterior wall 4740 of the cover 4700 form a channel 4770 to receive the diffusing member 4600. The slots 4760 allow flow through the cover 4700 while maintaining the diffusing member 4600 within the cover 4700 and minimizing contact, e.g., to prevent contamination.

The base member 4730 of the cover 4700 is engaged with the base member 4530 of the housing 4500 to secure the cover 4700 to the housing 4500 and support and retain the diffusing member 4600 to the housing 4500. The base member 4730 of the cover 4700 is engaged with the base member 4530 of the housing 4500 until the outer wall 4720 of the cover 4700 abuts the supports 4535/support members 4540 of the housing 4500, which supports 4535/support members 4540 space the cover 4700 from the housing 4500 to form the aforementioned lateral openings 4450.

Further, the spaced-apart slots 4760 of the cover 4700 are arranged to cover the diffusing member 4600 and the anterior ends of the outer openings 4560 of the vent and AAV housing 4500 so that at least a portion of the flow entering and/or exiting the outer openings 4560 can flow through the diffusing member 4600 and the spaced-apart slots 4760. Also, the inner openings 4750 of the cover 4700 are arranged to align with the opening 4610 of the diffusing member 4600 and the inner openings 4550 of the vent and AAV housing 4500 so that the inner openings 4750 of the cover 4700 are not covered by the diffusing member 4600 and flow entering and/or exiting the inner openings 4750 of the cover 4700 will flow directly through the inner openings 4550 of the housing 4500 and not flow into the diffusing member 4600.

The cover 4700 may be removably or permanently secured to the housing 4500 in any suitable manner, e.g., press fit assembly, snap or interference fit assembly, adhesive, ultrasonic welding, etc. In an example, the cover 4700 may be removably secured to the housing 4500, e.g., to allow cleaning and/or replacement of the diffusing material 4600. Alternatively, the cover 4700 may be permanently secured to the housing 4500 which may allow the entire vent and AAV assembly 4400 to be replaced, rather than replace the individual diffusing member 4600.

In the example of Figs. 5D to 5P, the cover 4700 includes one or more stakes 4780 adapted to engage within respective openings 4580 provided to the housing 4500 to secure the cover 4700 to the housing 4500, e.g., via ultrasonic weld.

In an alternative example, the vent and AAV assembly 4400 may be provided without a cover 4700, and the diffusing member 4600 may be secured to the housing 4500 in other suitable manners, e.g., diffusing member adhered or overmolded to the housing. In this example, the diffusing member 4600 may be more exposed, e.g., to facilitate drying. Also, such arrangement eliminates a component to reduce the overall size, e.g., height, of the vent and AAV assembly, e.g., lower profile.

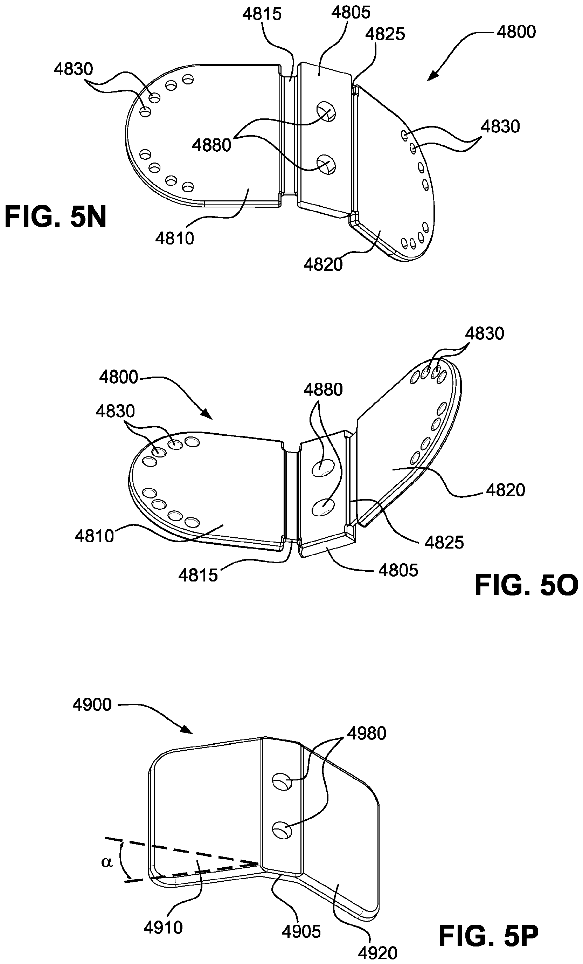

The AAV member 4800, e.g., see Figs. 5N and 5O, includes a dual-flap arrangement structured and arranged to selectively cover the inner openings 4550 and the outer openings 4560 of the housing 4500 in use. As illustrated, the AAV member 4800 includes a retaining portion 4805, a first flap portion 4810 that is movably connected, e.g., hingedly connected by a hinge portion 4815, to the retaining portion 4805 which allows the first flap portion 4810 to pivot relative to the retaining portion 4805, and a second flap portion 4820 that is movably connected, e.g., hingedly connected by a hinge portion 4825, to the retaining portion 4805 which allows the second flap portion 4820 to pivot relative to the retaining portion 4805. In an example, the hinge portions 4815, 4825 may be relatively large or thick to improve durability of the AAV member 4800. Each of the first and second flap portions 4810, 4820 includes a plurality of vent holes 4830 extending therethrough. As illustrated, the plurality of vent holes 4830 are arranged along the semi-circular end of each of the first and second flap portions 4810, 4820.

The AAV cover 4900, e.g., see Fig. 5P, is structured and arranged to maintain the AAV member 4800 to the vent and AAV housing 4500. As illustrated, the AAV cover 4900 includes a base member 4905, a first wall 4910 oriented at an angle to the base member 4905, and a second wall 4920 oriented at an angle to the base member 4905. In the illustrated example, each of the first and second walls 4910, 4920 forms an acute angle α with respect to the base member, e.g., 20°-60°, e.g., 30°-45°.

The retaining portion 4805 of the AAV member 4800 is arranged between the base member 4905 of the cover 4900 and the base member 4530 of the housing 4500, and the base member 4905 of the cover 4900 is engaged with the base member 4530 of the housing 4500 to secure the cover 4900 to the housing 4500 while sandwiching and retaining the AAV member 4800 to the housing 4500.

The AAV cover 4900 may be removably or permanently secured to the housing 4500 in any suitable manner, e.g., press fit assembly, snap or interference fit assembly, adhesive, ultrasonic welding, etc. In an example, the cover 4900 may be removably secured to the housing 4500, e.g., to allow cleaning and/or replacement of the AAV member 4800. Alternatively, the cover 4900 may be permanently secured to the housing 4500 which may allow the entire vent and AAV assembly 4400 to be replaced, rather than replace the individual AAV member 4800.

In the example of Figs. 5D to 5P, the cover 4700 includes one or more stakes 4780 adapted to extend through respective openings 4580 provided to the housing 4500, though respective openings 4880 provided to the retaining portion 4805 of the AAV member 4800, and into respective openings 4980 provided to the base member 4905 of the cover 4900 to secure the AAV member 4800 in position, e.g., via ultrasonic weld. This sandwiches and compresses the AAV member 4800 between the housing 4500 and the cover 4900 (e.g., see Fig. 5F).

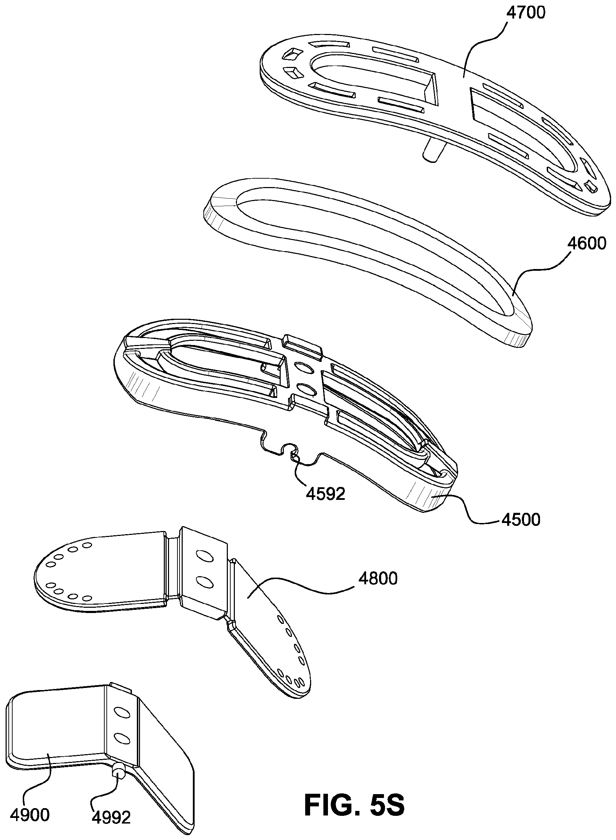

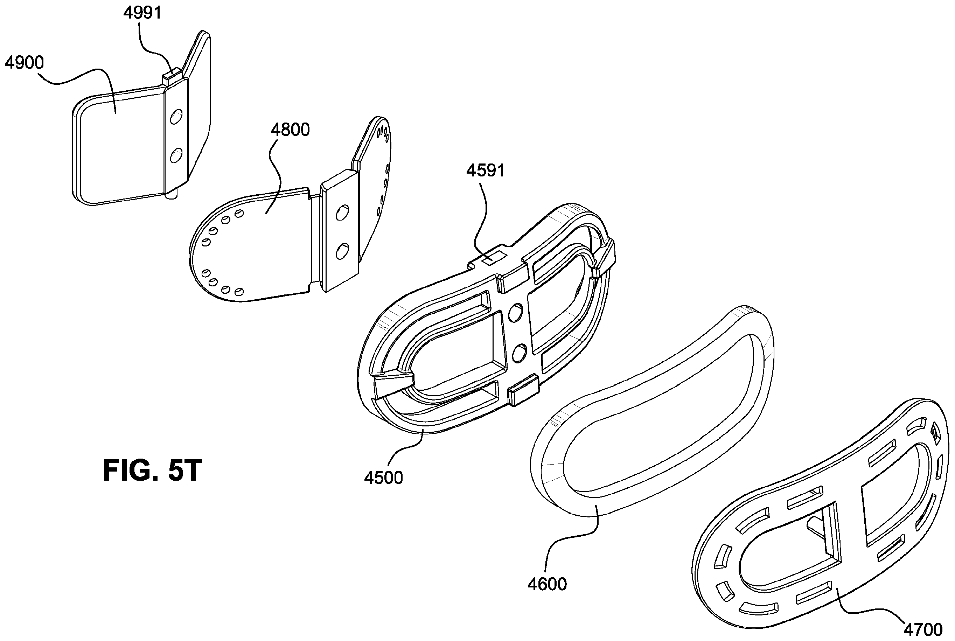

In example, the housing 4500 and the cover 4900 may comprise further retention features to secure the cover 4900 to the housing 4500. For example, as shown in Figs. 5S and 5T, one side of the cover 4900 may include a tab 4991 and the other side of the cover 4900 may include a pin 4992, the tab 4991 received within an opening 4591 in the housing 4500 and the pin 4992 engaged with a slotted opening 4592 in the housing, e.g., with a snap-fit, to further secure the cover 4900 to the housing 4500. However, it should be appreciated that other retention features and arrangements are possible.

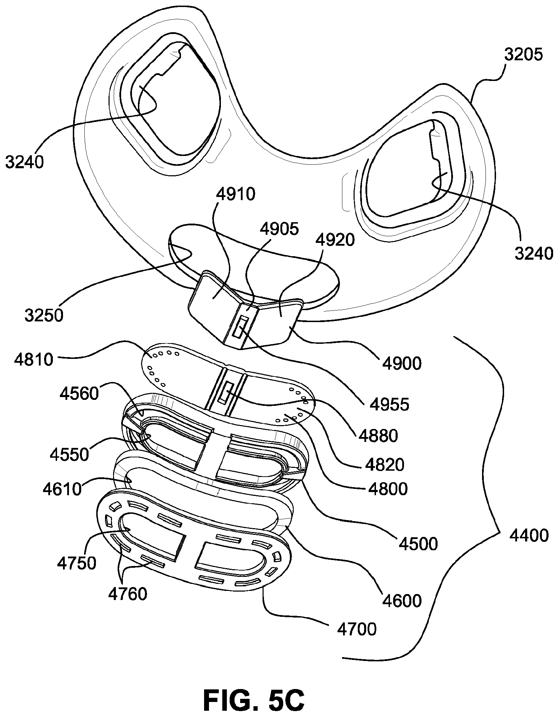

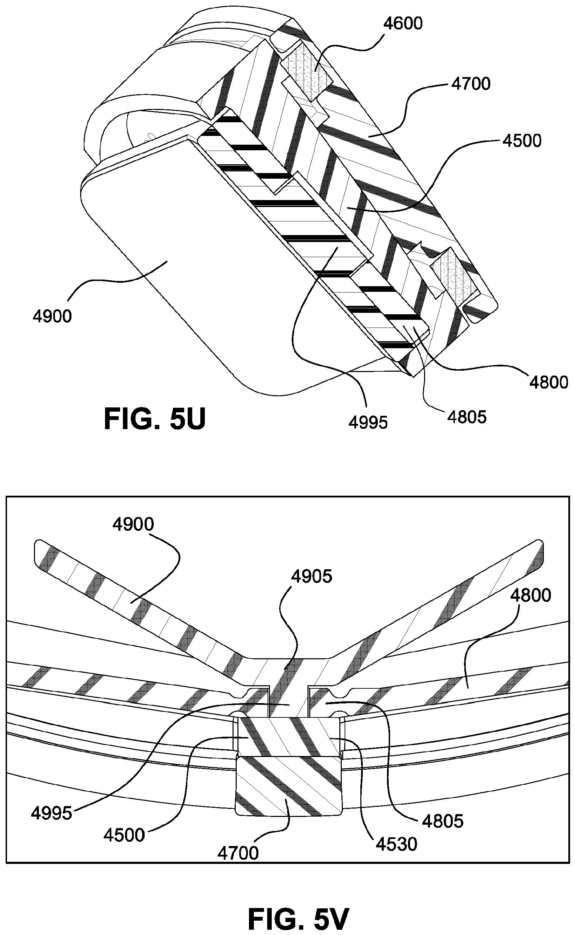

In the example of Figs. 5C, 5U, and 5V, the cover 4900 may include a protrusion 4995 that is adapted to extend through an opening 4580 provided to the AAV member 4800 and into engagement with the housing 4500 to secure the AAV member 4800 in position, e.g., via ultrasonic weld. This sandwiches and compresses the AAV member 4800 between the housing 4500 and the cover 4900. In such arrangement, the AAV member 4800 seals off the ultrasonic weld and the gap between the housing 4500 and the cover 4900, thereby ensuring that the ultrasonic weld does not cause any MPMU issues.

In the illustrated example, as shown in Fig. 5F, the first and second flap portions 4810, 4820 of the AAV member 4800 are biased or pre-loaded relative to the retaining portion 4805 into engagement with respective first and second walls 4910, 4920 of the cover 4900 which define stops for the first and second flap portions 4810, 4820. This arrangement allows the first and second flap portions 4810, 4820 to remain in a "rest" position when pressurized gas is not of sufficient magnitude or not delivered. As illustrated, the first and second walls 4910, 4920 are structured to cover or engage along an inner portion of respective first and second flap portions 4810, 4820. However, it should be appreciated that the first and second walls 4910, 4920 may be modified to cover or engage along larger or smaller portions of respective first and second flap portions 4810, 4820, e.g., the entirety of respective first and second flap portions 4810, 4820.

The AAV member 4800 is supported by the cover 4900 and the housing 4500 adjacent the posterior side of the vent and AAV housing 4500. The first and second flap portions 4810, 4820 are movable towards and away from the inner openings 4550 and the outer openings 4560 of the housing 4500, e.g., depending on the presence of pressurized gas.

The plurality of vent holes 4830 of the first and second flap portions 4810, 4820 are arranged to align with the outer openings 4560 of the housing 4500 so that the outer openings 4560 of the housing 4500 cannot be completely covered or closed by the first and second flap portions 4810, 4820 to allow flow through the outer openings 4560 of the housing 4500. Also, the first and second flap portions 4810, 4820 are arranged to overlap the inner openings 4550 of the housing 4500 so that the inner openings 4550 of the housing 4500 may be selectively covered or closed by the first and second flap portions 4810, 4820 to restrict flow through the inner openings 4550 of the housing 4500.

The AAV member 4800 provides a single component with two active segments, i.e., the first and second flap portions 4810, 4820 hinged in the middle to the retaining portion 4805. Such configuration provides a symmetrical arrangement to reduce the effect of gravity.

Since the AAV member 4800 is segmented into smaller first and second flap portions 4810, 4820 (rather than a single, larger flap portion), the working angle or hinge opening radius for each flap portion 4810, 4820 may be less. That is, the required opening for sufficient AAV performance may be split between the first and second flap portions 4810, 4820, so that each of the first and second flap portions 4810, 4820 may deflect a smaller angle to provide the required opening than a single, larger flap portion. This arrangement makes the vent and AAV assembly 4400 smaller, and provides a smaller profile as the flap portions 4810, 4820 protrude less into the breathing chamber of the patient interface when deactivated.

Further, since the AAV member 4800 is segmented into smaller first and second flap portions 4810, 4820 (rather than a single, larger flap portion), the AAV member 4800 provides less thumping effect when deactivating, i.e., less noise when the first and second flap portions 4810, 4820 move into engagement or contact with respective first and second walls 4910, 4920 of the cover 4900.

In an example, the AAV member 4800 (also referred to as an AAV membrane) may comprise a one-piece construction of a relatively flexible, elastic material, e.g., silicone or other thermoplastic elastomer. In another example, the AAV member 4800 may comprise a one-piece construction of a plastic material, e.g., polycarbonate. In another example, the AAV member 4800 may comprise a combination of elastic and plastic materials, e.g., first and second flap portions 4810, 4820 comprising a plastic material (e.g., polycarbonate) and the retaining portion 4805 and hinge portions 4815, 4825 comprising an elastic material (e.g., silicone). In each example, the plurality of vent holes 4830 in the first and second flap portions 4810, 4820 may be molded or laser cut. In an example, flap portions 4810, 4820 comprising a plastic material (e.g., polycarbonate) may provide better tolerances for the plurality of vent holes 4830.

In an alternative example, the gas washout vent provided to each of the flap portions 4810, 4820 may be a textile vent (e.g., one or a plurality of vent holes formed by interspaces between the fibers of a textile material) or a microvent (e.g., plurality of micro-sized vent holes (diameter of 1 micron or less) formed in a substrate of a semi-permeable material (e.g., using a laser drill or chemical etchant)). In an example, the textile vent or microvent may provide about 20-80 vent holes.

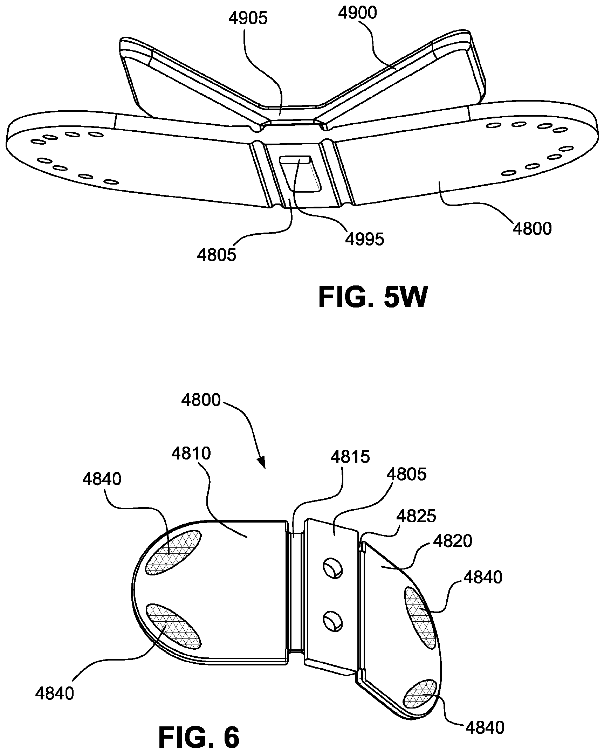

For example, Fig. 6 shows an AAV member 4800 with two textile or microvent vent portions 4840 provided to each of the flap portions 4810, 4820. In the illustrated example, the vent portions 4840 are arranged along the semi-circular end of each of the first and second flap portions 4810, 4820. However, it should be appreciated that the vent portions 4840 may be arranged along respective flap portions 4810, 4820 in other suitable manners. In addition, it should be appreciated that the number of vent portions (e.g., one or more vent portions per flap portion), size of each vent portion (e.g., area of each vent portion), and vent porosity (e.g., vent hole size) provided to each flap portion may be modified, e.g., depending on the desired gas washout rate.

In an example, the textile or microvent vent portions 4840 and respective flap portions 4810, 4820 may comprise an overmolded construction, i.e., elastic or plastic flap portions 4810, 4820 overmolded to the vent portions 4840. However, the vent portions 4840 may be provided to respective flap portions 4810, 4820 in other suitable manners, e.g., vent portions 4840 adhered or otherwise permanently secured to the respective flap portions 4810, 4820 in an operative position.

Further examples and details of textile vents or microvents are described in PCT Publication No. WO 2014/015382.

In an example, the housing 4500, the cover 4700, and the cover 4900 may be constructed (e.g., molded) of a relatively rigid material, e.g., thermoplastic polymer (e.g., polycarbonate). In an example, one or more portions of the vent and AAV assembly 4400 may be formed in one piece with the shell 3205, e.g., by over-molding or insert-molding. For example, the housing 4500 may be formed in one piece with the shell 3205.

Also, in an example, the AAV member 4800 may be formed in one piece with the housing 4500 or the cover 4900. For example, the AAV member 4800 (e.g., comprising silicone) may be overmolded to the housing 4500 (e.g., comprising polycarbonate) or to the cover 4900 (e.g., comprising polycarbonate). Fig. 5W shows an example of the AAV member 4800 overmolded to the cover 4900. This arrangement may comprise a more modular design and have less MPMU or cleaning issues.

The vent and AAV assembly 4400 is structured and arranged to regulate flow therethrough to (1) provide a vent flow path when pressure in the patient interface is above a predetermined magnitude and (2) provide a breathable flow path when pressure in the patient interface is below a predetermined magnitude or not delivered.

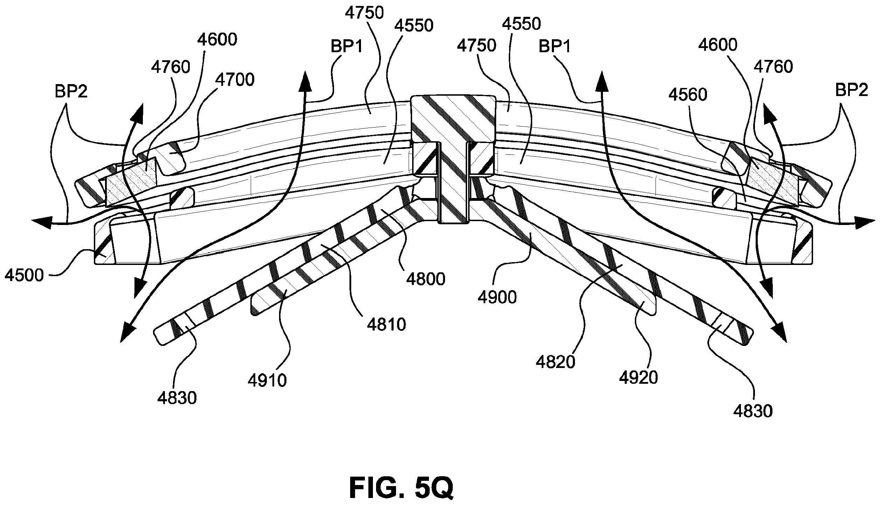

As shown in Fig. 5Q, when pressure in the patient interface is below a predetermined magnitude or not delivered (e.g., when the RPT device is not operating), the first and second flap portions 4810, 4820 of the AAV member 4800 assume a rest or deactivated position so that air may pass through the inner and outer openings 4550, 4560 of the housing 4500. That is, pressurized gas is not delivered to the patient interface or is not of sufficient magnitude which allows the first and second flap portions 4810, 4820 to be biased or deflected downwardly away from the the housing 4500 and the inner and outer openings 45550, 4560 and into engagement with respective first and second walls 4910, 4920 of the AAV cover 4900. As a result, air may pass through primary breathable flow paths BP1 that extend through the inner openings 4550 of the housing 4500, through the opening 4610 of the diffusing member, and through the inner openings 4750 of the cover 4700. In addition, air may pass through secondary breathable flow paths BP2 that extend through the outer openings 4560 of the housing 4500, through the diffusing member 4600 and the slots 4760 of the cover 4700, and through the lateral openings 4450 between the cover 4700/diffusing member 4600 and the housing 4500. Such primary and secondary breathable flow paths BP1, BP2 provide air paths to allow the patient to breathe in ambient air and exhale.

While air may potentially flow though the diffusing member 4600 as noted above, it should be appreciated that AAV performance will not be effected by the diffusing member 4600. That is, the diffusing member 4600 does not change the flow direction and has very low impact on flow dynamics and impedance. The vent and AAV assembly 4400 provides an arrangement to allow air to directly enter and exit the patient interface and reduce CO₂ retention, e.g., via the primary breathable flow paths BP1.

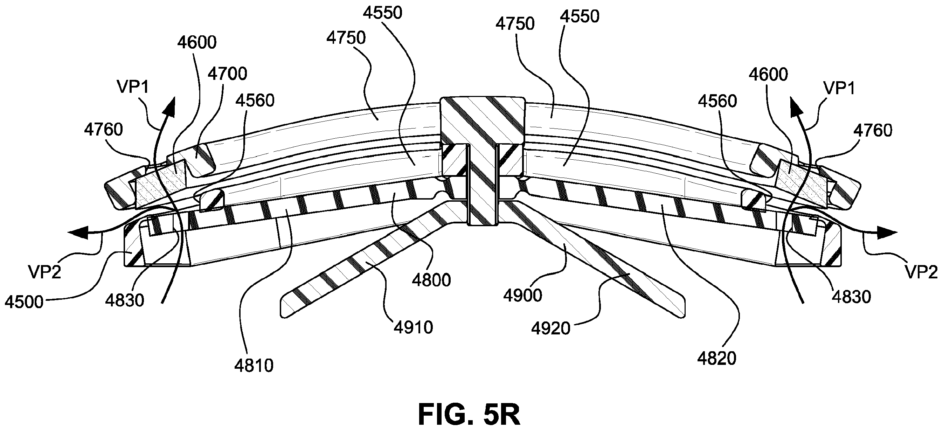

As shown in Fig. 5R, when pressure in the patient interface is above a predetermined magnitude (e.g., when the RPT device is operating), the increase in pressure within the pressurized volume of the patient interface creates a pressure gradient or vacuum which draws or sucks the first and second flap portions 4810, 4820 into an activated position so that air may only pass though the plurality of vent holes 4830 in the first and second flap portions 4810, 4820 and the outer openings 4560 of the housing 4500. That is, pressure in the patient interface is of sufficient magnitude to force and maintain the first and second flap portions 4810, 4820 in engagement with the posterior side of the housing 4500. As a result, the plurality of vent holes 4830 in the first and second flap portions 4810, 4820 are arranged to align with the outer openings 4560 of the housing 4500 so that air may pass through primary vent flow paths VP1 that extend through the vent holes 4830 of the first and second flap portions 4810, 4820, through the outer openings 4560 of the housing 4500, through the diffusing member 4600, and through the slots 4760 of the cover 4700. In addition, air may pass through secondary vent flow paths VP2 that extend through the vent holes 4830 of the first and second flap portions 4810, 4820, through the outer openings 4560 of the housing 4500, and through the lateral openings 4450 between the cover 4700/diffusing member 4600 and the housing 4500. Such primary and secondary vent flow paths VP1, VP2 provide vent paths to allow sufficient gas washout to prevent CO₂ build-up in the patient interface. The primary vent flow path VP 1 directs flow through the diffusing member 4600 to atmosphere to diffuse the exhaust vent flow to produce less noise. The secondary vent flow path VP2 provides an alternative path that bypasses the diffusing member 4600, e.g., to allow sufficient gas washout when the diffusing member 4600 is blocked due to water or humidity.

Aspects of the vent and AAV assembly 4400 may be tuned to provide a desired flow curve within a therapeutic pressure range. In an example, venting characteristics of each of components may be tuned, e.g., based on venting requirement, sound requirement, treatment requirement, etc. This arrangement allows a more customized vent and AAV assembly for the patient.

For example, the shape, size, and number of vent holes 4830 through the first and second flap portions 4810, 4820 may be tuned to regulate vent flow along the primary and secondary vent flow paths VP1, VP2. For example, the vent holes 4830 of the first and second flap portions 4810, 4820 form an inlet for exhaust flow along the primary and secondary vent flow paths VP1, VP2, and the size of each vent hole (e.g., cross-sectional area of each hole) may be tuned to provide the desired flow curve within a therapeutic pressure range, e.g., a substantially constant flow rate over a therapeutic pressure range. In an example, the oronasal patient interface of the type shown in Fig. 4 provides more deadspace in the headgear tubes 3340, which may require a larger vent, i.e., larger number and/or size vent holes.

With respect to the diffusing member 4600, the thickness and material of the diffusing member 4600 may be tuned to regulate flow. Also, the shape, size, and number of slots 4760 through the cover 4700 may be tuned to regulate flow.

### 5.3.5 Decoupling structure(s)

In one form the patient interface 3000 includes at least one decoupling structure, for example, a swivel or a ball and socket.

### 5.3.6 Connection port

Connection port 3600 allows for connection to the air circuit 4170.

### 5.3.7 Forehead support

In one form, the patient interface 3000 includes a forehead support 3700.

### 5.3.8 Anti-asphyxia valve

In one form, the patient interface 3000 includes an anti-asphyxia valve.

### 5.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block of the RPT device 4000 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller. One example of an air circuit 4170 comprising a heated wire circuit is described in United States Patent 8,733,349.

### 5.4.1 Supplementary gas delivery

In one form of the present technology, supplementary gas, e.g. oxygen, may be delivered to one or more points in the pneumatic path, such as upstream of the pneumatic block, to the air circuit 4170, and/or to the patient interface 3000.

### 5.5 GLOSSARY

For the purposes of the present technology disclosure, in certain forms of the present technology, one or more of the following definitions may apply. In other forms of the present technology, alternative definitions may apply.

### 5.5.1 General

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. `Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmHzO, g-f/cm² and hectopascal. 1 cmHzO is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmHzO.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator.* A mechanical device that provides pressure support to a patient to perform some or all of the work of breathing.

### 5.5.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.5.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions.

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmHzO pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.5.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) *Flattened:* Having a rise followed by a relatively flat portion, followed by a fall.
(ii) *M-shaped:* Having two local peaks, one at the leading edge, and one at the trailing edge, and a relatively flat portion between the two peaks.
(iii) *Chair-shaped*: Having a single local peak, the peak being at the leading edge, followed by a relatively flat portion.
(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve*.

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.5.3 Ventilation

*Adaptive Servo-Ventilator (ASV)*: A servo-ventilator that has a changeable, rather than fixed target ventilation. The changeable target ventilation may be learned from some characteristic of the patient, for example, a respiratory characteristic of the patient.

*Backup rate:* A parameter of a ventilator that establishes the minimum breathing rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP)*: a base pressure, to which a pressure varying within the breath is added to produce the desired interface pressure which the ventilator will attempt to achieve at a given time.

*End expiratory pressure (EEP):* Desired interface pressure which the ventilator will attempt to achieve at the end of the expiratory portion of the breath. If the pressure waveform template Π(Φ) is zero-valued at the end of expiration, i.e. Π(Φ) = 0 when Φ = 1, the EEP is equal to the EPAP.

*Inspiratory positive airway pressure (IPAP)*: Maximum desired interface pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure (e.g., *PS* = *IPAP* - *EPAP*)*.* In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous*/*Timed (SIT):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator delivers a breath of *air* to a spontaneously breathing patient, it is said to be triggered to do so at the initiation of the respiratory portion of the breathing cycle by the patient's efforts.

### 5.5.4 Anatomy

### 5.5.4.1 Anatomy of the face

*Ala:* the external outer wall or "wing" of each nostril (plural: alar)

### Alar angle:

*Alare:* The most lateral point on the nasal *ala.*

*Alar curvature (or alar crest) point.* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

### Lip, lower (labrale inferius):

### Lip, upper (labrale superius):

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton*: The point of greatest concavity in the midline of the lower lip between labrale inferius and soft tissue pogonion.

### 5.5.4.2 Anatomy of the skull

*Frontal bone:* The frontal bone includes a large vertical portion, the *squama frontalis*, corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.5.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.5.5 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent*: (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.5.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

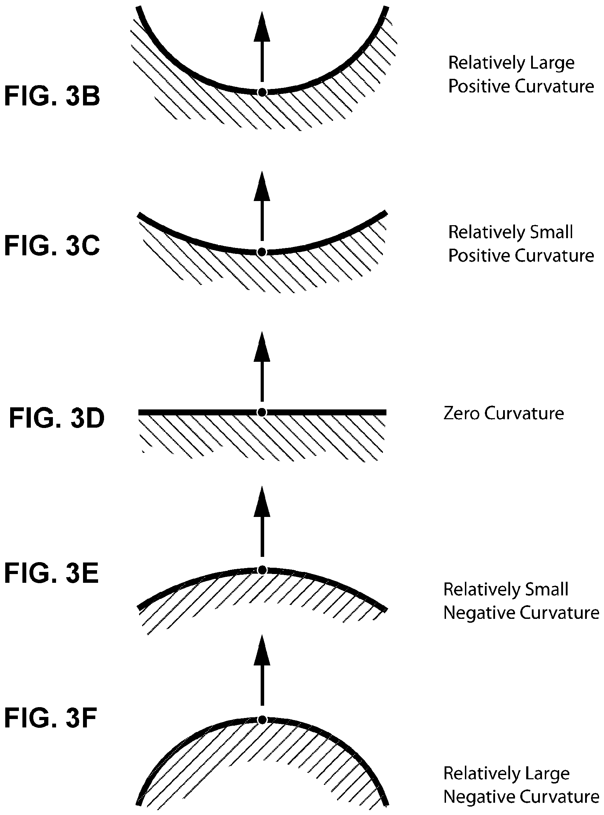

To facilitate describing the shape of the three-dimensional structures and the surfaces, we first consider a cross-section through a surface of the structure at a point, *p*. See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 5.5.6.1 Curvature in one dimension

The curvature of a plane curve at *p* may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 5.5.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures atp are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, `path length' will be taken to mean the distance along the surface from *f*(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance `as the crow flies'.)

### 5.5.6.3 Space curves

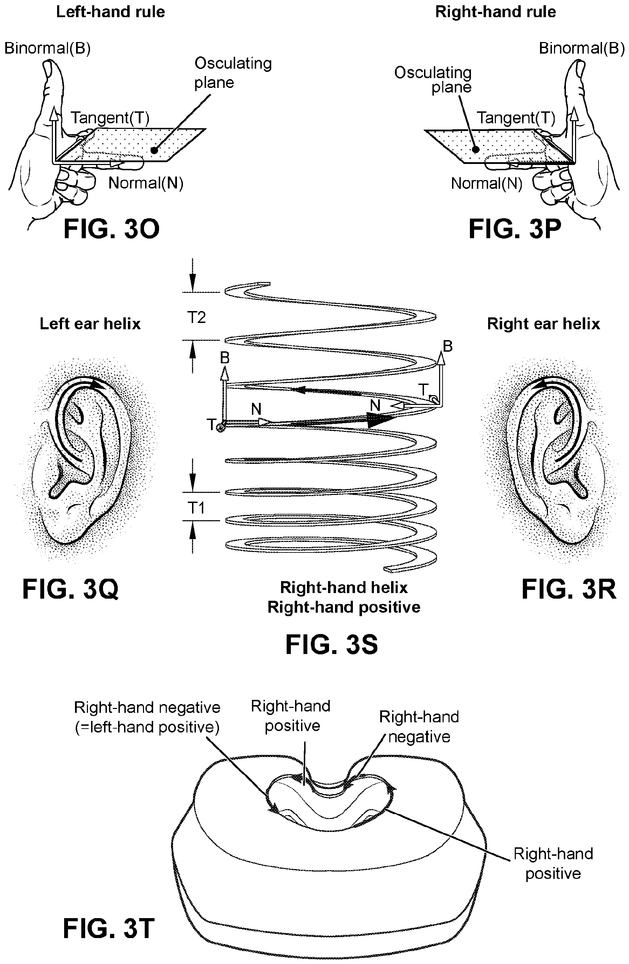

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.5.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.6 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 5.7 REFERENCE SIGNS LIST

| **Feature Item** | **Number** |
|---|---|
| patient | 1000 |
| bed partner | 1100 |
| patient interface | 3000 |
| seal - forming structure | 3100 |
| plenum chamber | 3200 |
| shell | 3205 |
| chord | 3210 |
| superior point | 3220 |
| inferior point | 3230 |
| inlet port | 3240 |
| opening | 3250 |
| positioning and stabilising structure | 3300 |
| upper strap | 3310 |
| lower strap | 3320 |
| connection point | 3325 |
| clip | 3326 |
| headgear tube | 3340 |
| tab | 3342 |
| headgear tube connector | 3344 |
| conduit headgear inlet | 3390 |
| vent | 3400 |
| connection port | 3600 |
| forehead support | 3700 |
| RPT device | 4000 |
| air circuit | 4170 |
| vent and AAV assembly | 4400 |
| lateral opening | 4450 |
| vent and AAV housing | 4500 |
| outer wall | 4510 |
| inner wall | 4520 |
| base member | 4530 |
| support | 4535 |
| support member | 4540 |
| inner opening | 4550 |
| outer opening | 4560 |
| opening | 4580 |
| opening | 4591 |
| opening | 4592 |
| diffusing member | 4600 |
| opening | 4610 |
| diffusing member cover | 4700 |
| outer wall | 4710 |
| inner wall | 4720 |
| base member | 4730 |
| anterior wall | 4740 |
| inner opening | 4750 |
| slot | 4760 |
| channel | 4770 |
| stake | 4780 |
| AAV member | 4800 |
| retaining portion | 4805 |
| first flap portion | 4810 |
| hinge portion | 4815 |
| second flap portion | 4820 |
| hinge portion | 4825 |
| vent hole | 4830 |
| vent portion | 4840 |
| opening | 4880 |
| AAV cover | 4900 |
| base member | 4905 |
| first wall | 4910 |
| second wall | 4920 |
| opening | 4980 |
| tab | 4991 |
| pin | 4992 |
| protrusion | 4995 |
| humidifier | 5000 |

## Claims

1. A patient interface (3000) to deliver a flow of air at a positive pressure with respect to ambient air pressure to an entrance to the patient's airways including at least the entrance of a patient's nares while the patient is sleeping, to ameliorate sleep disordered breathing, the patient interface comprising:
a seal-forming structure (3100) constructed and arranged to form a seal with a region of a patient's face surrounding the entrance to the patient's airways, the seal-forming structure (3100) forming at least a portion of a plenum chamber (3200) pressurizable to a therapeutic pressure; and
a vent and Anti-asphyxia-valve (AAV) assembly (3850) configured to regulate flow therethrough to (1) provide a vent flow path (VP1, VP2) when pressure in the plenum chamber (3200) is above a predetermined magnitude and (2) provide a breathable flow path (BP1, BP2) when pressure in the plenum chamber (3200) is below the predetermined magnitude or not delivered,
the vent and AAV assembly (3850) comprising:
a vent and AAV housing (4500) including at least one first opening (4550) and at least one second opening (4560) extending therethrough, each of the at least one first opening (4550) and the at least one second opening (4560) configured to allow gas to flow between the plenum chamber and ambient; and
an AAV member (4800) provided to the vent and AAV housing (4500), the AAV member (4800) including at least one flap portion (4810, 4820) structured and arranged to regulate flow through the at least one first opening (4550) and the at least one second opening (4560),
wherein the at least one flap portion (4810, 4820) includes a plurality of vent holes (4830) therethrough, the plurality of vent holes (4830) being arranged to align with the at least one second opening (4560),
wherein the at least one flap portion (4810, 4820) is movable to an activated position when pressure in the plenum chamber (3200) is above the predetermined magnitude to cover the at least one first opening (4550) and the at least one second opening (4560) so that a vent flow of gas is allowed to pass along the vent flow path (VP1, VP2) that extends through the plurality of vent holes (4830) of the at least one flap portion (4810, 4820) and through the at least one second opening (4560), and
wherein the at least one flap portion (4810, 4820) is movable to a deactivated position when pressure in the plenum chamber (3200) is below the predetermined magnitude or not delivered to uncover the at least one first opening (4550) and the at least one second opening (4560) so that a breathable flow of gas is allowed to pass along the breathable flow path (BP1, BP2) that extends through the at least one first opening (4550) and the at least one second opening (4560).

2. The patient interface according to claim 1, further comprising a diffusing member (4800) provided to the vent and AAV housing (4500), the diffusing member (4800) configured and arranged such that the at least one second opening (4560) is covered by the diffusing member (4800) so that at least a portion of the vent flow of gas passes through the diffusing member (4800) along the vent flow path (VP1, VP2) and the at least one first opening (4550) is not covered by the diffusing member (4800) so that at least a portion of the breathable flow of gas bypasses the diffusing member (4800) along the breathable flow path (BP1, BP2).

3. The patient interface according to claim 2, wherein the diffusing member (4800) comprises a textile material.

4. The patient interface according to any one of claims 2 to 3, wherein the diffusing member (4800) includes at least one opening (4610) arranged to align with the at least one first opening (4550) of the vent and AAV housing (4500).

5. The patient interface according to any one of claims 2 to 4, further comprising a diffusing member cover (4700) to retain the diffusing member (4800) to the vent and AAV housing (4500).

6. The patient interface according to any one of claims 1 to 5, further comprising an AAV cover (4900) to retain the AAV member (4800) to the vent and AAV housing (4500).

7. The patient interface according to claim 6, wherein the AAV cover (4900) forms a stop for the at least one flap portion (4810, 4820) when in the deactivated position.

8. The patient interface according to any one of claims 1 to 7, the AAV member (4800) includes a first flap portion (4810) and a second flap portion (4820), each of the first and second flap portions including the plurality of vent holes (4830).

9. The patient interface according to any one of claims 1 to 8, wherein at least the at least one flap portion (4810, 4820) comprises a plastic material.

10. The patient interface according to any one of claims 1 to 9, wherein the AAV member (4800) comprises a silicone material.

11. The patient interface according to any one of claims 1 to 10, wherein the at least one flap portion (4810, 4820) includes a textile vent or a microvent providing the plurality of vent holes (4830).

12. The patient interface according to claim 11, wherein the at least one flap portion (4810, 4820) includes the textile vent, and wherein the textile vent comprises a textile material with the plurality of vent holes (4830) formed by interspaces between the fibers of the textile material.

13. The patient interface according to claim 11, wherein the at least one flap portion (4810, 4820) includes the microvent, and wherein the microvent comprises a semi-permeable material with the plurality of vent holes (4830) formed in a substrate of the semi-permeable material, and each of the plurality of vent holes (4830) includes a diameter of 1 micron or less.

14. A Continuous Positive Airway Pressure (CPAP) system for providing gas at positive pressure for respiratory therapy to a patient (1000), the CPAP system comprising:
an RPT device (4000) configured to supply a flow of gas at a therapeutic pressure;
a patient interface (3000) according to any one of claims 1 to 13; and
an air delivery conduit configured to pass the flow of gas at the therapeutic pressure from the RPT device (4000) to the patient interface (3000).

## Patentansprüche

1. Patientenschnittstelle (3000) zur Abgabe einer Luftströmung mit einem Überdruck im Hinblick auf den Umgebungsluftdruck zu einem Eingang zu den Patientenluftwegen, darunter mindestens einem Eingang der Nasenlöcher eines Patienten, während der Patient schläft, um schlafbezogene Atmungsstörungen zu mildern, wobei die Patientenschnittstelle aufweist:
eine dichtungsbildende Struktur (3100), die so aufgebaut und angeordnet ist, dass sie eine Dichtung mit einer Region des Gesichts eines Patienten bildet, die den Eingang zu den Patientenluftwegen umgibt, wobei die dichtungsbildende Struktur (3100) mindestens einen Abschnitt einer Plenumkammer (3200) bildet, die auf einen Therapiedruck druckbeaufschlagbar ist; und
eine Entlüftungs- und Anti-Asphyxieventil- (AAV) Anordnung (3850), die so konfiguriert ist, dass sie eine Strömung durch sie hindurch reguliert, um (1) einen Entlüftungsströmungsweg (VP1, VP2) bereitzustellen, wenn der Druck in der Plenumkammer (3200) über einer vorbestimmten Größe liegt, und (2) einen Atemströmungsweg (BP1, BP2) bereitzustellen, wenn der Druck in der Plenumkammer (3200) unter der vorbestimmten Größe liegt oder nicht abgegeben wird,
wobei die Entlüftungs- und AAV-Anordnung (3850) aufweist:
ein Entlüftungs- und AAV-Gehäuse (4500) mit mindestens einer ersten Öffnung (4550) und mindestens einer zweiten Öffnung (4560), die sich dadurch erstrecken, wobei die mindestens eine erste Öffnung (4550) und die mindestens eine zweite Öffnung (4560) jeweils so konfiguriert sind, dass sie Gas zwischen der Plenumkammer und der Umgebung strömen lassen; und
ein AAV-Teil (4800), das am Entlüftungs- und AAV-Gehäuse (4500) vorgesehen ist, wobei das AAV-Teil (4800) mindestens einen Klappenabschnitt (4810, 4820) aufweist, der so strukturiert und angeordnet ist, dass er die Strömung durch die mindestens eine erste Öffnung (4550) und die mindestens eine zweite Öffnung (4560) reguliert,
wobei der mindestens eine Klappenabschnitt (4810, 4820) mehrere durchgehende Entlüftungslöcher (4830) aufweist, wobei die mehreren Entlüftungslöcher (4830) so angeordnet sind, dass sie zu der mindestens einen zweiten Öffnung (4560) ausgerichtet sind,
wobei der mindestens eine Klappenabschnitt (4810, 4820) in eine aktivierte Position beweglich ist, wenn der Druck in der Plenumkammer (3200) über der vorbestimmten Größe liegt, um die mindestens eine erste Öffnung (4550) und die mindestens eine zweite Öffnung (4560) zu bedecken, so dass eine Entlüftungsgasströmung entlang des Entlüftungsströmungswegs (VP1, VP2) passieren kann, der sich durch die mehreren Entlüftungslöcher (4830) des mindestens einen Klappenabschnitts (4810, 4820) und durch die mindestens eine zweite Öffnung (4560) erstreckt, und
wobei der mindestens eine Klappenabschnitt (4810, 4820) in eine deaktivierte Position beweglich ist, wenn der Druck in der Plenumkammer (3200) unter der vorbestimmten Größe liegt oder nicht abgegeben wird, um die mindestens eine erste Öffnung (4550) und die mindestens eine zweite Öffnung (4560) freizulegen, so dass eine Atemgasströmung entlang des Atemströmungswegs (BP1, BP2) passieren kann, der sich durch die mindestens eine erste Öffnung (4550) und die mindestens eine zweite Öffnung (4560) erstreckt.

2. Patientenschnittstelle nach Anspruch 1, die ferner ein Verteilungsteil (4800) aufweist, das am Entlüftungs- und AAV-Gehäuse (4500) vorgesehen ist, wobei das Verteilungsteil (4800) so konfiguriert und angeordnet ist, dass die mindestens eine zweite Öffnung (4560) durch das Verteilungsteil (4800) bedeckt ist, so dass mindestens ein Anteil der Entlüftungsgasströmung das Verteilungsteil (4800) entlang des Entlüftungsströmungswegs (VP1, VP2) durchläuft, und die mindestens eine erste Öffnung (4550) nicht durch das Verteilungsteil (4800) bedeckt ist, so dass mindestens ein Anteil der Atemgasströmung das Verteilungsteil (4800) entlang des Atemströmungswegs (BP1, BP2) umgeht.

3. Patientenschnittstelle nach Anspruch 2, wobei das Verteilungsteil (4800) ein Textilmaterial aufweist.

4. Patientenschnittstelle nach einem der Ansprüche 2 bis 3, wobei das Verteilungsteil (4800) mindestens eine Öffnung (4610) aufweist, die so angeordnet ist, dass sie zu der mindestens einen ersten Öffnung (4550) des Entlüftungs- und AAV-Gehäuses (4500) ausgerichtet ist.

5. Patientenschnittstelle nach einem der Ansprüche 2 bis 4, die ferner eine Verteilungsteilabdeckung (4700) aufweist, um das Verteilungsteil (4800) am Entlüftungs- und AAV-Gehäuse (4500) festzuhalten.

6. Patientenschnittstelle nach einem der Ansprüche 1 bis 5, die ferner eine AAV-Abdeckung (4900) aufweist, um das AAV-Teil (4800) am Entlüftungs- und AAV-Gehäuse (4500) festzuhalten.

7. Patientenschnittstelle nach Anspruch 6, wobei die AAV-Abdeckung (4900) einen Anschlag für den mindestens einen Klappenabschnitt (4810, 4820) in der deaktivierten Position bildet.

8. Patientenschnittstelle nach einem der Ansprüche 1 bis 7, wobei das AAV-Teil (4800) einen ersten Klappenabschnitt (4810) und einen zweiten Klappenabschnitt (4820) aufweist, wobei der erste und zweite Klappenabschnitt jeweils die mehreren Entlüftungslöcher (4830) aufweisen.

9. Patientenschnittstelle nach einem der Ansprüche 1 bis 8, wobei mindestens der mindestens eine Klappenabschnitt (4810, 4820) ein Kunststoffmaterial aufweist.

10. Patientenschnittstelle nach einem der Ansprüche 1 bis 9, wobei das AAV-Teil (4800) ein Silikonmaterial aufweist.

11. Patientenschnittstelle nach einem der Ansprüche 1 bis 10, wobei der mindestens eine Klappenabschnitt (4810, 4820) eine Textilentlüftung oder eine Mikroentlüftung aufweist, die die mehreren Entlüftungslöcher (4830) vorsieht.

12. Patientenschnittstelle nach Anspruch 11, wobei der mindestens eine Klappenabschnitt (4810, 4820) die Textilentlüftung aufweist und wobei die Textilentlüftung ein Textilmaterial mit den mehreren Entlüftungslöchern (4830) aufweist, die durch Zwischenräume zwischen den Fasern des Textilmaterials gebildet sind.

13. Patientenschnittstelle nach Anspruch 11, wobei der mindestens eine Klappenabschnitt (4810, 4820) die Mikroentlüftung aufweist und wobei die Mikroentlüftung ein halbdurchlässiges Material mit den mehreren Entlüftungslöchern (4830) aufweist, die in einem Substrat des halbdurchlässigen Materials gebildet sind, und die mehreren Entlüftungslöcher (4830) jeweils einen Durchmesser von höchstens 1 Mikrometer aufweisen.

14. CPAP-System (Continuous Positive Airway Pressure system) zur Bereitstellung von Gas mit Überdruck zur Atemtherapie eines Patienten (1000), wobei das CPAP-System aufweist:
eine RPT-Vorrichtung (4000), die so konfiguriert ist, dass sie eine Gasströmung mit einem Therapiedruck zuführt;
eine Patientenschnittstelle (3000) nach einem der Ansprüche 1 bis 13; und
eine Luftabgabeleitung, die so konfiguriert ist, dass sie die Gasströmung mit dem Therapiedruck von der RPT-Vorrichtung (4000) zur Patientenschnittstelle (3000) führt.

## Revendications

1. Interface patient (3000) destinée à administrer un flux d'air à une pression positive par rapport à une pression d'air ambiante à une entrée des voies respiratoires du patient comprenant au moins une entrée des narines du patient pendant que le patient dort, de façon à améliorer une respiration de sommeil perturbée, l'interface patient comprenant :
une structure de formation d'étanchéité (3100) structurée et conçue pour former une étanchéité avec une région du visage d'un patient entourant l'entrée des voies respiratoires du patient, la structure de formation d'étanchéité (3100) formant au moins une partie d'une chambre de distribution (3200) pouvant être pressurisée à une pression thérapeutique ; et
un ensemble évent et valve anti-asphyxie (AAV) (3850) conçu pour réguler un flux à travers ce dernier pour (1) fournir un trajet de flux d'évent (VP1, VP2) lorsqu'une pression dans la chambre de distribution (3200) est supérieure à une amplitude prédéterminée et pour (2) fournir un trajet de flux pouvant être respiré (BP1, BP2) lorsqu'une pression dans la chambre de distribution (3200) est inférieure à l'amplitude prédéterminée, ou n'est pas administrée,
l'ensemble évent et AAV (3850) comprenant :
un boîtier d'évent et d'AAV (4500) comprenant au moins une première ouverture (4550) et au moins une seconde ouverture (4560) s'étendant à travers ce dernier, chacune de l'au moins une première ouverture (4550) et de l'au moins une seconde ouverture (4560) étant conçue pour permettre à du gaz de circuler entre la chambre de distribution et l'environnement ambiant ; et
un élément AAV (4800) disposé dans le boîtier d'évent et d'AAV (4500), l'élément AAV (4800) comprenant au moins une partie clapet (4810, 4820) structurée et conçue pour réguler un écoulement à travers l'au moins une première ouverture (4550) et l'au moins une seconde ouverture (4560) ,
dans laquelle l'au moins une partie clapet (4810, 4820) comprend une pluralité de trous d'évent (4830) la traversant, les trous de la pluralité de trous d'évent (4830) étant conçus pour s'aligner avec l'au moins une seconde ouverture (4560),
dans laquelle l'au moins une partie clapet (4810, 4820) est mobile vers une position activée lorsqu'une pression dans la chambre de distribution (3200) est supérieure à l'amplitude prédéterminée pour couvrir l'au moins une première ouverture (4550) et l'au moins une seconde ouverture (4560) de sorte qu'un flux d'évent de gaz puisse passer le long du trajet de flux d'évent (VP1, VP2) qui s'étend à travers la pluralité de trous d'évent (4830) de l'au moins une partie clapet (4810, 4820) et à travers l'au moins une seconde ouverture (4560), et
dans laquelle l'au moins une partie clapet (4810, 4820) est mobile vers une position désactivée lorsqu'une pression dans la chambre de distribution (3200) est inférieure à l'amplitude prédéterminée ou n'est pas administrée pour découvrir l'au moins une première ouverture (4550) et l'au moins une seconde ouverture (4560) de sorte qu'un flux de gaz pouvant être respiré puisse passer le long du trajet de flux pouvant être respiré (BP1, BP2) qui s'étend à travers l'au moins une première ouverture (4550) et l'au moins une seconde ouverture (4560).

2. Interface patient selon la revendication 1, comprenant en outre un élément de diffusion (4800) prévu dans le boîtier d'évent et d'AAV (4500), l'élément de diffusion (4800) étant conçu et disposé de sorte que l'au moins une seconde ouverture (4560) soit couverte par l'élément de diffusion (4800) de sorte qu'au moins une partie du flux de gaz d'évent traverse l'élément de diffusion (4800) le long du trajet de flux d'évent (VP1, VP2) et que l'au moins une ouverture (4550) ne soit pas couverte par l'élément de diffusion (4800) de sorte qu'au moins une partie du flux de gaz pouvant être respiré contourne l'élément de diffusion (4800) le long du trajet de flux pouvant être respiré (BP1, BP2).

3. Interface patient selon la revendication 2, dans laquelle l'élément de diffusion (4800) comprend une matière textile.

4. Interface patient selon l'une ou l'autre des revendications 2 ou 3, dans laquelle l'élément de diffusion (4800) comprend au moins une ouverture (4610) disposée pour s'aligner avec l'au moins une première ouverture (4550) du boîtier d'évent et d'AAV (4500).

5. Interface patient selon l'une quelconque des revendications 2 à 4, comprenant en outre un couvercle d'élément de diffusion (4700) servant à retenir l'élément de diffusion (4800) par rapport au boîtier d'évent et d'AAV (4500).

6. Interface patient selon l'une quelconque des revendications 1 à 5, comprenant en outre un couvercle d'AAV (4900) servant à retenir l'élément AAV (4800) par rapport au boîtier d'évent et d'AAV (4500).

7. Interface patient selon la revendication 6, dans laquelle le couvercle d'AAV (4900) forme une butée destinée à l'au moins une partie clapet (4810, 4820) lorsqu'elle est dans la position désactivée.

8. Interface patient selon l'une quelconque des revendications 1 à 7, dans laquelle l'élément AAV (4800) comprend une première partie clapet (4810) et une seconde partie clapet (4820), chacune des première et seconde parties clapets comprenant la pluralité de trous d'évent (4830) .

9. Interface patient selon l'une quelconque des revendications 1 à 8, dans laquelle au moins l'au moins une partie clapet (4810, 4820) comprend une matière plastique.

10. Interface patient selon l'une quelconque des revendications 1 à 9, dans laquelle l'élément AAV (4800) comprend un matériau silicone.

11. Interface patient selon l'une quelconque des revendications 1 à 10, dans laquelle l'au moins une partie clapet (4810, 4820) comprend un évent en textile ou un micro-évent fournissant la pluralité de trous d'évent (4830) .

12. Interface patient selon la revendication 11, dans laquelle l'au moins une partie clapet (4810, 4820) comprend l'évent en textile, et dans laquelle l'évent en textile comprend une matière textile dotée de la pluralité de trous d'évent (4830) formés par des interstices entre les fibres de la matière textile.

13. Interface patient selon la revendication 11, dans laquelle l'au moins une partie clapet (4810, 4820) comprend le micro-évent, et dans laquelle le micro-évent comprend un matériau semi-perméable dotée de la pluralité de trous d'évent (4830) formés dans un substrat du matériau semi-perméable, et chaque trou de la pluralité de trous d'évent (4830) a un diamètre inférieur ou égal à 1 micron.

14. Système de pression positive continue des voies respiratoires (CPAP) destiné à fournir un gaz à une pression positive de thérapie respiratoire à un patient (1000), le système de CPAP comprenant :
un dispositif de RPT (4000) conçu pour délivrer un flux de gaz à une pression thérapeutique ;
une interface patient (3000) selon l'une quelconque des revendications 1 à 13 ; et
un conduit d'administration d'air conçu pour faire passer le flux de gaz à la pression thérapeutique du dispositif de RPT (4000) à l'interface patient (3000).
